(19) Europäisches Patentamt
European Patent Office
Office européen des brevets

(11) Numéro de publication: **0 236 163**
**B1**

# (12) FASCICULE DE BREVET EUROPEEN

(45) Date de publication du fascicule du brevet: **03.10.90**

(21) Numéro de dépôt: **87400149.8**

(22) Date de dépôt: **22.01.87**

(51) Int. Cl.$^5$: **C 07 C 311/19,** C 07 K 5/06, A 61 K 37/02

## (54) Dérivés N alpha-substitués des N alpha-arylsulfonylaminoacyl p-amidinophénylalaninamides, leur préparation, leur application comme médicaments et intermédiaires pour leur synthèse.

(30) Priorité: **24.01.86 FR 8601398**
**24.01.86 FR 8601400**

(43) Date de publication de la demande:
**09.09.87 Bulletin 87/37**

(45) Mention de la délivrance du brevet:
**03.10.90 Bulletin 90/40**

(84) Etats contractants désignés:
**AT BE CH DE ES FR GB GR IT LI LU NL SE**

(56) Documents cités:

**CHEMICAL ABSTRACTS, vol. 98, 1983, page 645, résumé no. 107770b, Columbus, Ohio, US**
**CHEMICAL ABSTRACTS, vol. 102, 1985, page 735, résumé no. 25017y, Columbus, Ohio, US; B. VOIGT et al.: "Synthesis of Nalpha-(arylsulfonylglycylglycyl)-4-amidinophenylalanine amides as thrombin inhibitors"**

(73) Titulaire: **SANOFI**
**40, Avenue George V**
**F-75008 Paris (FR)**

(72) Inventeur: **Delabassee, Denis**
**276 Chemin des Crêtes**
**Goyrans F-31120 Portet/Garonne (FR)**
Inventeur: **Bernat, André**
**14 Chemin Maurens**
**F-31270 Cugnaux (FR)**
Inventeur: **Maffrand, Jean-Pierre**
**5 rue du Corps Franc Pommies**
**F-31120 Portet/Garonne (FR)**
Inventeur: **Vallee, Eric**
**253 Chemin du Ramelet Moundi**
**F-31170 Tournefeuille (FR)**
Inventeur: **Frehel, Daniel**
**Résidence l'Autan Appt. 206**
**100 Allée de Barcelone§F-31000 Toulouse (FR)**

(74) Mandataire: **Bressand, Georges et al**
**c/o CABINET LAVOIX 2 Place d'Estienne d'Orves**
**F-75441 Paris Cédex 09 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**EP 0 236 163 B1**

(56) Documents cités:

CHEMICAL ABSTRACTS, vol. 97, 1982, page 232, résumé no. 105943r, Columbus, Ohio, US; J. STUERZEBECHER et al.: "Are synthetic inhibitors able to improve factor Xa assays using peptide substrates"

DIE PHARMAZIE, vol. 40, no. 5, mai 1985, pages 305-306, VEB Verlag Volk und Gesundheit, Berlin, DE; B. VOIGT et al.. "Synthese vom Nalpha-Benzyloxycarbonyl-4-amidinophenylalaninamiden als Thrombininhibitoren"

**Description**

La présente invention est relative à de nouveaux dérivés Nα-substitués des Nα-arylsulfonylaminoacyl p-amidino-phénylalaninamides, à leur procédé de préparation, et à leur utilisation en tant qu'agents inhibiteurs sélectifs de la thrombine et antithrombotiques.

Les composés de l'invention répondent à la formule générale (I):

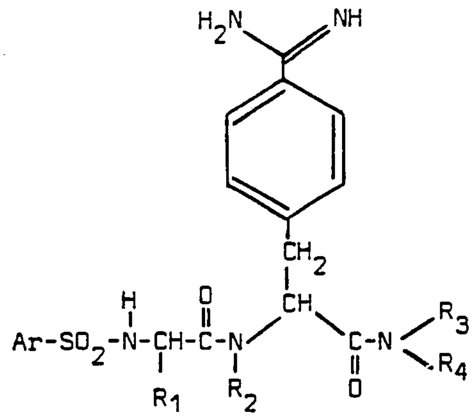

(I)

dans laquelle:

$R_1$ représente l'hydrogène, un groupe alcoyle ou hydroxyalcoyle en $C_1$—$C_6$, benzyle, un groupe phényle ou un groupe hydroxy-4 phényle;

$R_2$ représente un groupe alcoyle, alcényle ou alcynyle ayant jusqu'à 6 atomes de carbone, ou un groupe benzyle, ou un groupe alcoxy en ($C_1$—$C_6$) carbonylalcoyle en $C_1$—$C_6$, carboxyalcoyle en $C_1$—$C_6$ ou hydroxyalcoyle en $C_1$—$C_6$;

$R_3$ et $R_4$, identiques ou différents, représentent chacun un radical alcoyle, hydroxyalcoyle, alcényle ou alcynyle ayant jusqu'à 6 atomes de carbone, ou forment ensemble, avec l'azote auquel ils sont attachés, un hétérocycle saturé choisi parmi morpholino, thiomorpholino, pyrrolidino non substitué ou substitué par un groupe alcoxy ($C_1$—$C_6$) carbonyle ou carboxy, pipérazino, (alcoyle en $C_1$—$C_6$)-4 pipérazino, (hydroxyalcoyle en $C_1$—$C_6$)-4 pipérazino, ou pipéridino non substitué ou substitué par un groupe alcoyle en $C_1$—$C_6$ benzyle, hydroxy, hydroxyalcoyle en $C_1$—$C_6$, amino, aminoalcoyle en $C_1$—$C_6$ hydroxyamino, alcoxy en ($C_1$—$C_6$) carbonyle ou carboxy;

Ar représente un groupe phényle, alpha-naphthyle, béta-naphtyle, méthoxycarbonyl-phényle, ou bien un groupe hétéroaryle choisi parmi les radicaux pyridyle, quinoléinyle, isoquinoléinyle ou methyl-quinoléinyle.

Les composés de formule (I), ci-dessus préférés, sont ceux dans lesquels $R_1$ représente l'hydrogène ou un radical alcoyle, ceux dans lesquels $R_2$ représente un radical alcoyle, ceux dans lesquels le groupe

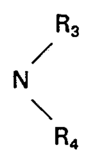

représente un radical pipéridino substitué par un groupe methyle ou benzyle ou non substitué et ceux dans lesquels Ar représente un radical naphthyle.

Dans le cas où $R_1$ est autre que l'hydrogène, le carbone porteur du groupe $R_1$, comme celui du groupe phénylalanine, peut avoir la configuration R ou S ou RS et pour ces derniers composés, la cristallisation peut entraîner un enrichissement de certains des diastéréoisomères. Tous les composés présentant les dites configurations sont compris dans la présente invention.

Les composés de formule (I) ci-dessus, comportant un ou plusieurs centres asymétriques peuvent exister sous formes de plusieurs isomères (diastéréoisomères, énantiomères) qui peuvent être préparés par synthèse stéréospécifique ou séparés de leurs homologues par des moyens classiques. L'invention concerne aussi des sels d'addition des composés de formule (I) avec les acides minéraux ou organiques pharmaceutiquement acceptables.

Le termes "alcoyle inférieur", "alcényle inférieur", et "alcynyle inférieur" tels qu'utilisés ici, désignent les radicaux d'hydrocarbures aliphatiques ramifiés ou linéaires, contenant jusqu'à 6 atomes de carbone tels que méthyle, éthyle, isopropyle, isobutyle, tertiobutyle, n-hexyle, allyle, propargyle, crotyle, méthyl-2 crotyle, méthyl-2 allyle, butyryle-2.

Des inhibiteurs de thrombine synthétiques, présentant un groupe amidinophénylalamine ont été décrits dans la littérature.

G. WAGNER et ses collaborateurs (DD Patent 142807 (16.7.80)) ont décrit des composés de formule générale (A):

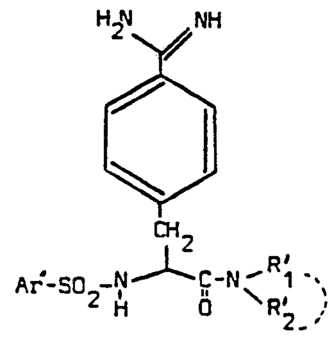

(A)

L'insertion d'un résidu aminoacide glycine entre le groupe sulfonyle et l'azote N-alpha de la p-amidinophénylalamine a conduit à des composés de formule générale (B), dont l'activité in vitro est potentialisée par rapport à ceux de formule générale (A) (G. WAGNER et Coll.DD Patent 155954 (3.2.81)).

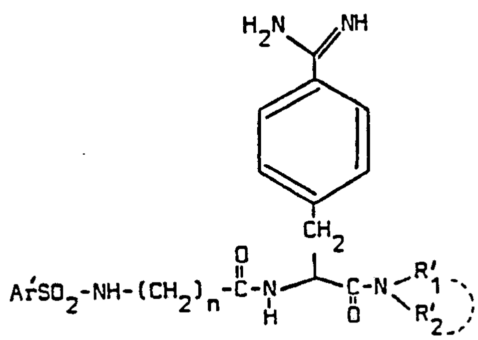

(B)

et parmi ceux-ci, le composé de formule (B) où n = 1, A'r = béta-naphtyle, $NR'_1R'_2$ = pipéridino, ci-après désigné composé (C) présente la meilleure activité inhibitrice de la thrombine in vitro (J. STURZEBECHER et al. Thrombosis Research, 1983, *29*, 635) et ex vivo (J. HAUPTMANN et al. Thrombosis Research, 1985, *39*, 771).

Les composés de formule générale (A) et (B) ci-dessus sont préparés selon les procédés décrits dans les brevets DD 142804 et DD 155954, les amides étant obtenues à partir des acides libres correspondants par activation et réaction avec l'amine correspondante. Ces procédés impliquent des conditions de réactions qui induisent des racémisations au niveau du centre asymétrique; en outre, ils ne permettent pas d'obtenir des composés portant le substituant $R_2$.

La demanderesse a trouvé que les composés de formule (I) ci-dessus peuvent être obtenus par un procédé qui permet, par l'utilisation de procédés de couplages et de groupements protecteurs judicieusement choisis, de respecter les centres d'asymétrie dans leur configuration originelle, et qui n'induit pas de racémisation.

Ce résultat est obtenu, contrairement aux procédés décrits par G. WAGNER et ses collaborateurs, en construisant d'abord la partie amide

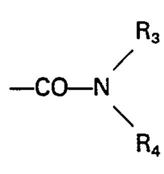

à partir de la fonction acide du synthon p-cyanophénylalanine, avant la partie arylsulfonylaminoacyle, afin de pouvoir introduire facilement le substituant $R_2$.

L'invention a également pour objet un procédé de préparation des composés de formule (I) caractérisé en ce que l'on fait réagir sur la cyano-4 phénylalaninamide Nα-alcoylée de formule (II)

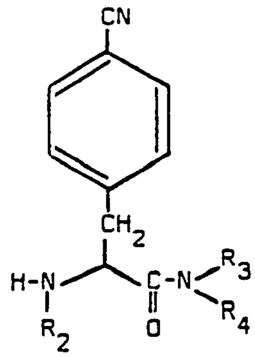

(II)

dans laquelle $R_2$, $R_3$ et $R_4$ ont les mêmes significations que dans la formule (I), un acide de formule:

$$Ar—SO_2—NH—\underset{\displaystyle R_1}{\underset{|}{CH}}—COOH \qquad (III)$$

sous sa forme activée:

$$Ar—SO_2—NH—\underset{\displaystyle R_1}{\underset{|}{CH}}—CO—R \qquad (IV)$$

dans laquelle Ar et $R_1$ ont les mêmes significations que dans la formule (I) et R représente un bon groupement nucléofuge, tel que chloro, alcoxycarbonyloxy ou hétéroaryle, pour obtenir le composé de formule (V):

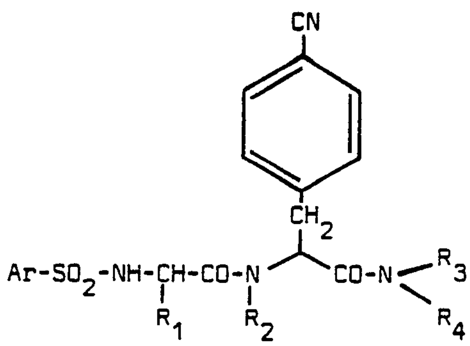

(V)

dans laquelle Ar, $R_1$, $R_2$, $R_3$ et $R_4$ ont les mêmes significations que dans la formule (I), qu'on traite avec un excès d'une solution saturée de gaz chlorhydrique dans un alcool de formule X—OH dans laquelle X représente un radical alcoyle inférieur, pour obtenir le composé de formule (VI) sous forme de chlorhydrate.

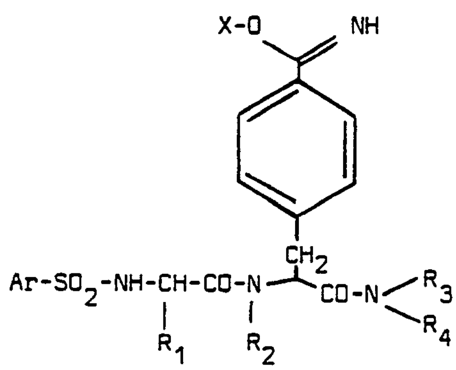

(VI)

dans lequel Ar, $R_1$, $R_2$, $R_3$, $R_4$ et X ont les mêmes significations précitées. L'imidoester de formule (VI) est alors traité par un excès d'une solution de gaz ammoniac dans un alcool inférieur à la température d'ébullition du mélange réactionnel pour obtenir le composé de formule (I) recherché.

Ce composé est isolé sous forme de sel, le base libre pouvant être obtenue par les procédés classiques et éventuellement transformée en un autre sel pharmaceutiquement acceptable tel que par exemple, outre le chlorhydrate, le bromhydrate, le sulfate, le méthanesulfonate, le naphtalènesulfonate-2, le maléate, le fumarate, le citrate, l'acétate, le gluconate, le dobésilate, le sultosilate.

Le préparation du nouveau composé de formule (II) s'effectue à partir de la cyano-4 phénylalanine de formule:

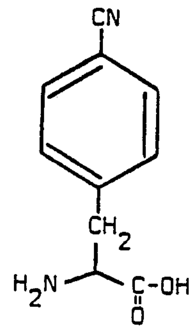

L'acide de formule:

$$Ar—SO_2—NH—CH—COOH \quad (III)$$
$$|$$
$$R_1$$

a été préparé selon le schéma réactionnel:

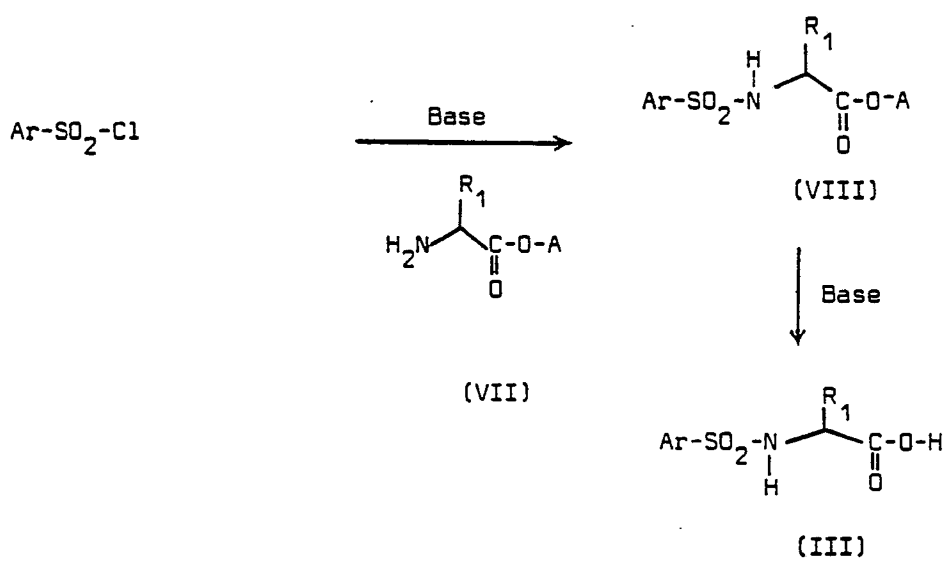


(VII) (VIII) (III)

L'introduction d'un centre asymétrique dans l'aminoester (VII, A = alcoyle inférieur) dont la configuration "R" ou "S" initiale doit être conservée jusqu'à l'acide (III), nécessite l'emploi de méthodes non racémisantes; telles que par exemple:

la sulfonylation de l'aminoester (VII) s'opère en milieu biphasique de préférence le mélange eau-dichlorométhane, eau-chloroforme, eau-tétrachlorure de carbone, en présence d'une base, de préférence un carbonate alcalin tel que le carbonate de potassium, le carbonate de sodium, à des températures comprises entre 10°C et 25°C.

la saponification de l'ester (VIII) s'opère en milieu hydroalcoolique tel que eau-méthanol, ou eau-éthanol, en présence d'un équivalent d'hydroxyde alcalin, de préférence l'hydroxyde de sodium, à des températures comprises entre 10°C et 25°C. La neutralisation du milieu réactionnel par addition d'un équivalent d'une solution aqueuse 1N d'acide minéral, de préférence l'acide chlorhydrique, conduit à l'acide (III). Cette saponification peut également être menée à bien dans un milieu hydro-organique, tel que eau-dioxanne dans les mêmes conditions.

Pour la transformation de l'acide de formule (III) en ester activé de formule (IV), 2 cas sont à envisager:

a) *Cas où $R_1 = H$*, et ceux dans lesquels le problème de racémisation de l'acide (III) n'existe pas (méthode non stéréospécifique)

On peut utiliser indifféremment l'activation de la fonction acide du synthon par exemple par:

transformation de la fonction acide en halogénure d'acyle (IV): R = Cl)

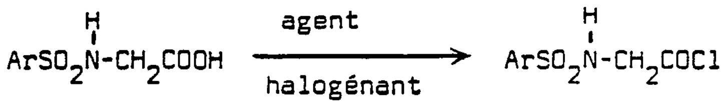


(III : $R_1$ = H) (IV : R = Cl ; $R_1$ = H)

selon le procédé décrit dans le brevet DDR 155954.

transformation de la fonction acide en anhydride carbonique mixte (IV):

$R = O-C(=O)-OY_1$) selon le schéma réactionnel:

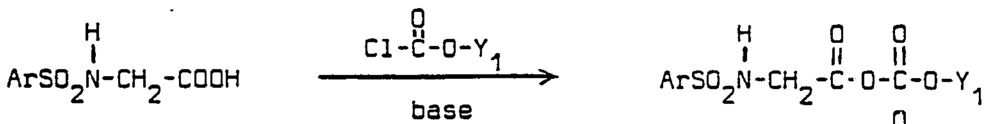


(III : $R_1$=H) (IV : R = $O-C(=O)-O-Y_1$ ; $R_1$ = H)

La réaction utilise un chloroformate d'alcoyle

$$Cl-CO-Y_1,$$

où $Y_1$ est un radical alcoyle inférieur ramifié ou non, en présence d'une amine tertiaire comme base. Le chloroformate d'alcoyle préférentiellement utilisé est le chloroformate d'éthyle ($Y_1 = C_2H_5$) ou d'isobutyle ($Y_1 = CH_2-CH(CH_3)_2$). L'amine tertiare préférentielle est la triéthylamine. Cette condensation s'opère de préférence à des températures comprises entre −5°C et +10°C, dans un solvant inerte tel que le dichlorométhane, la chloroforme ou le tétrachlorure de carbone.

b) *Cas où $R_1 \neq H$*: Lorsqu'on veut éviter une racémisation au niveau du carbone porteur du substituant $R_1$ (méthode stéréospécifique)

La transformation de la fonction acide des composés de formule (III) en esters activés, conduit à des composés de formule générale (IV: R = O—Ƶ) selon le schéma réactionnel:

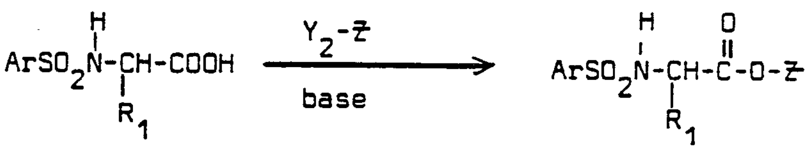


(III) (IV : R = O-Ƶ)

Les réactifs de couplage $Y_2$—Ƶ, n'induisant pas de racémisation, utilisés de préférence, mais non limitatifs, sont les suivants:

Hydroxy-1 benzotriazole (HOBT)

($Y_2$ = OH; Ƶ = 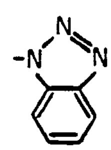 )

en présence de N,N-dicyclohexylcarbodiimide (DCC) selon le mode opératoire décrit par E. C. JORGENSEN et al. (J. Am. Chem. Soc. 1971, *93*, 6318).

Hexafluorophosphate de benzotriazoyl-1 oxytris (diméthylamino) phosphonium (BOP)

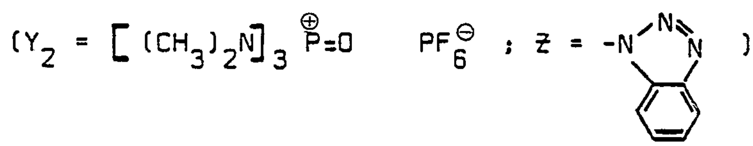

selon le mode opératoire décrit par B. CASTRO et al. (Synthesis 1976,751).

Chlorure de N,N-bis (oxo-2 oxazolidinyl-3) phosphorodiamidique

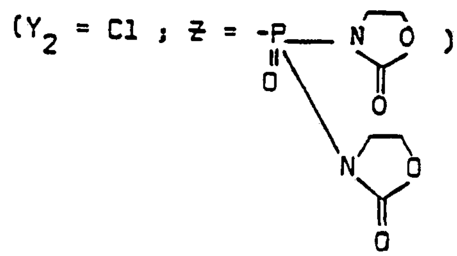

selon le mode opératoire décrit par D. H. RICH et al. (J. Am. Chem. Soc. 1985, *107*, 4342).

Les réactions d'activation et de couplage s'opèrent en présence d'amines tertiaires de préference la triéthylamine, dans un solvant inerte tel que le dichlorométhane, le diméthylformamide ou l'acétonitrile, à des températures comprises entre 15°C et 40°C.

La formation de l'imidoester (VI) s'effectue en milieu alcoolique tel que le méthanol ou l'éthanol, à une température comprise entre −10°C et +10°C, de préférence à 0°C pendant une durée de 16 H à 24 H.

L'amidine de formule (I) peut-être obtenue en traitant le composé (VI) précédemment obtenu, sans autre purification par une solution alcoolique de gaz ammoniac à une normalité de 3 N à 15 N, à la température ambiante, et on chauffe ensuite le mélange au reflux pendant 1 à 3 H.

Les composés de formule (II) ci-dessus, comportant un centre asymétrique, peuvent exister sous forme de deux isomères (énantiomères). L'invention concerne aussi bien chaque stéréoisomère que leurs mélanges. L'invention comprend aussi des sels d'addition avec les acides minéreaux ou organiques.

La présente invention concerne également un procédé de préparation des composés de formule (II) caractérisé en ce que l'on fait réagir l'acide aminé de formule (IX) sous sa forme activée (X):

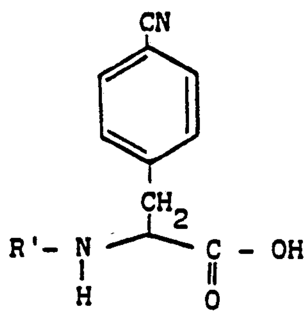

(IX)

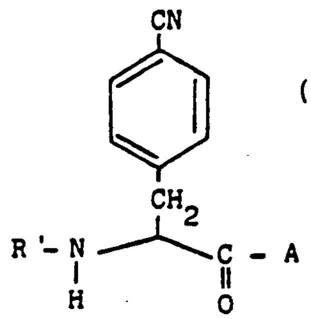

(X)

dans lesquelles R' représente un groupement N-protecteur et A représente le reste d'un réactif de couplage, avec l'amine de formule (XI):

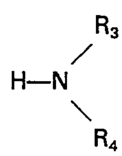

dans laquelle $R_3$ et $R_4$ sont tels que décrits dans la formule (II), pour former le composé de formule (XII):

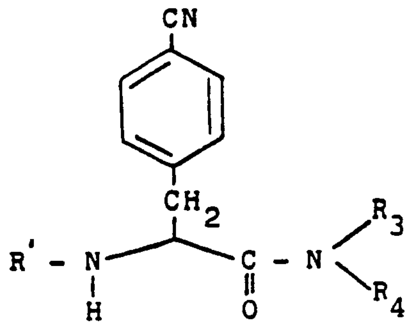

(XII)

dans laquelle R', $R_3$ et $R_4$ ont les significations précitées, qui, par action du composé $R_2X$ dans lequel X est un halogène tel que le chlore, le brome ou l'iode et $R_2$ est tel que défini dans la formule (II), conduit au composé de formule (XIII):

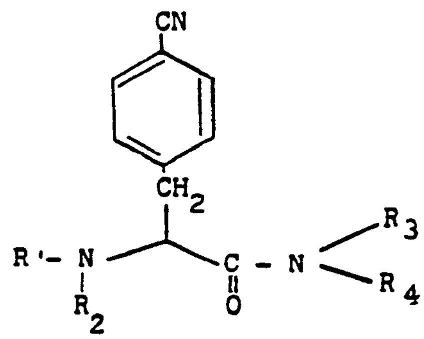

(XIII)

et par clivage du groupement protecteur R', on obtient les composés de formule (II).

La formation du composé de formule (IX) est obtenue par fixation du groupement N-protecteur R' sur la p-cyanophénylalanine de formule:

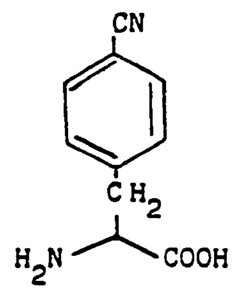

Le groupe N-protecteur, représenté par R', est un des groupements stables en milieu alcalin, utilisés pour la protection des groupes amino des acides aminés dans la chimie des peptides, par exemple le groupe ter-butyloxy carbonyle, de préférence désigné ci-après Boc; le groupe (diméthoxy-3,5 phényl)-2 propyl-2 oxycarbonyle désigné comme Ddz; le groupe (biphényl-4yl)-2 propyl-2 oxycarbonyle désigné comme Bpoc; le groupe (nitro-2 phényl)sulfényle désigné comme Nps.

Pour obtenir l'acide activé de formule (X) dans lequel A représente le reste du réactif de couplage, deux cas sont à envisager.

a) *Procédé de préparation avec conservation de la configuration "R" ou "S"* (synthèse stéréospécifique)

Pour ne pas induire de racémisation au niveau du centre asymétrique du composé de formule (XII) et conserver la configuration initiale du centre asymétrique de l'acide de formule (IX), il est nécessaire

d'employer une activation de l'acide (IX), utilisant la transformation de la fonction acide en ester activé (X) suivant le schéma réactionnel:

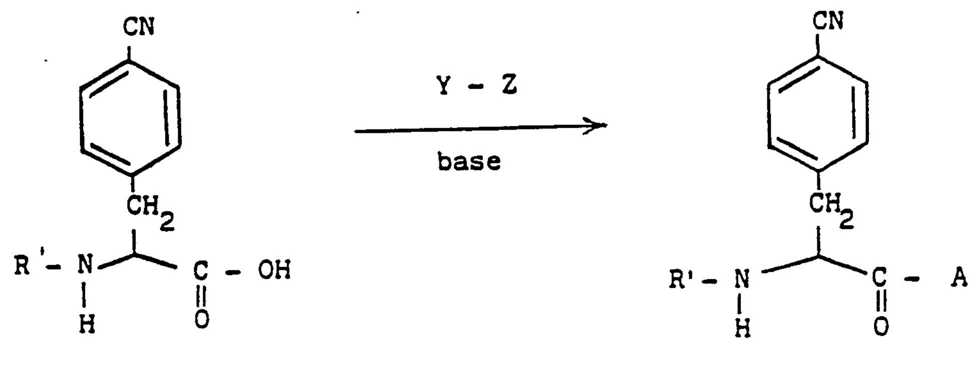


(IX) ( X)

Les réactifs de couplage Y—Z, n'induisant pas de racémisation, utilisés de préférence, mais non limitatifs, sont les suivants:

Hydroxy-1 benzotriazole (HOBT)

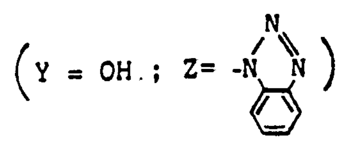

en présence de N,N-dicyclohexylcarbodiimide (DCC) selon le mode opératoire décrit par E. C. JORGENSEN et al. (J. Am. Chem. Soc. 1971, *93*, 6318).

Hexafluorophosphate de benzotriazolyl-1 oxytris (diméthylamino) phosphonium (BOP)

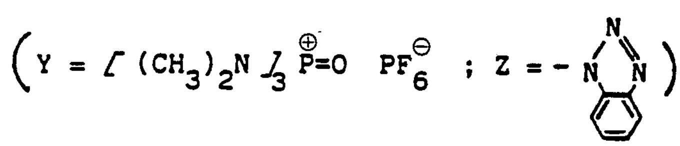

selon le mode opératoire décrit par B. CASTRO et al. (Synthesis 1976, 751).

Chlorure de N,N-bis (oxo-2 oxazolidinyl-3) phosphorodiamidique

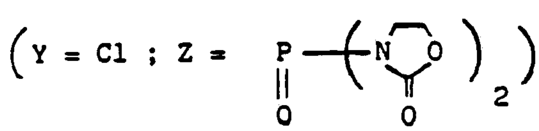

selon le mode opératoire décrit par D. H. RICH et al. (J. Am. Chem. Soc. 1985, *107*, 4342).

La réaction d'activation et de couplage s'opère en présence d'amines tertaires, de préférence la triéthylamine, dans un solvant inerte tel que le dichlorométhane, le diméthylformamide ou l'acétonitrile, à des températures comprises entre 15°C et 40°C.

b) *Procédé de préparation sans conservation de la configuration* (synthèse non stéréospécifique)

On peut effectuer l'activation de la fonction acide du composé (IX) par transformation de la fonction acide en anhydride carbonique mixte

(X: A = $-O-\underset{\underset{O}{\|}}{C}-O-B$) selon le schéma réactionnel:

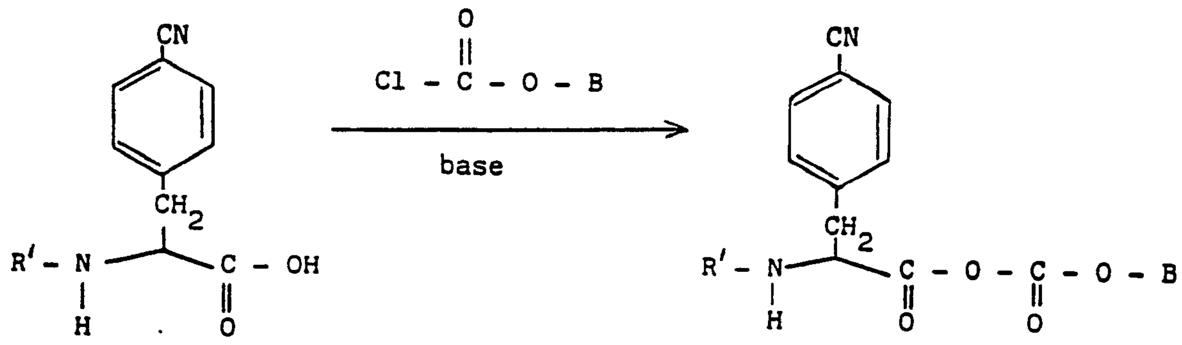


(IX) (X)

La réaction utilise un chloroformate d'alcoyle 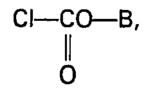

où B est un alcoyle inférieur ramifié ou non, en présence d'une amine tertaire comme base. Le chloroformate d'alcoyle préférentiellement utilisé est le chloroformate d'éthyle ($B = C_2H_5$) ou d'isobutyle ($B = CH_2—CH(CH_3)_2$).

L'amine tertiaire préférentielle est la triéthylamine. Cette condensation s'opère de préférence à des températures comprises entre −5°C et +10°C, dans un solvant inerte tel que le dichlorométhane, le chloroforme ou le tétrachlorure de carbone.

La p-cyanophénylalanine de départ a été préparée selon une des méthodes utilisées dans la littérature (G. WAGNER et al. Pharmazie 1981, *36* (9), 597).

Le composé de formule (X) est mis à réagir avec l'amine de formule (XI) dans un solvant inerte et en présence d'une amine tertiaire.

L'alcoylation du composé XII utilise un des méthodes classiques employées en chimie organique, par action de l'halogénure d'alcoyle $R_2—X$, dans lequel $R_2$ est tel que désigné ci-dessus dans la formule II, et X est un halogène tel que le chlore, le brome ou l'iode de préférence.

Cette opération s'effectue en présence d'une base forte telle qu'un hydrure alcalin, de préférence l'hydrure de sodium dans un solvant inerte tel que le diméthylformamide ou le tétrahydrofuranne, ou bien un alkyllithium tel que le butyllithium ou un amidure de lithium tel que le diisopropylamidure de lithium dans un solvant inerte tel que l'hexane ou le tétrahydrofuranne, à des températures comprise entre 0° et 20°C.

Le clivage du groupe N-protecteur R' du composé de formule (XIII) conduit aux p-cyanophénylalanin-amides Nα-substituées de formule (II). Ce clivage s'opère en milieu acide, de préférence un mélange acide bromhydrique-acide acétique ou bien dans l'acide trifluoroacétique (R' = Boc, Ddz, Bpoc, Nps), dans l'acide acétique (R' = Boc, Nps), dans une solution saturée de gaz chlorhydrique dans l'acétate d'éthyle (R' = Boc), à des températures comprises entre 0° et 20°C.

## Exemple 1

*Nα-(terbutyloxycarbonyl) p-cyanophénylalanine*

(IX: R' = 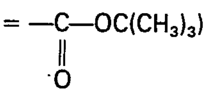)

On dissout 10 g (0,044 mole) de chlorhydrate de p-cyanophénylalanine dans 220 ml de dioxanne et 88,2 ml (0,088) mole) d'hydroxyde de sodium aqueux 1N. A température ambiante et sous atmosphère inerte, on ajoute par portions, au milieu réactionnel, 1,77 g (0,044 mole) d'oxyde de magnésium, puis 10,6 g (0,0484 mole) de dicarbonate de diterbutyle. On agite à température ambiante, pendant 20 h. On filtre les cristaux qui sont lavés avec de l'eau. On évapore le filtrat et dissout le résidu dans l'eau. La phase aqueuse obtenue est amenée à pH = 3 par addition d'une solution saturée d'hydrogénosulfate de potassium. On extrait la phase aqueuse avec 2 × 400 ml d'acétate d'éthyle, sèche les extraits organiques sur sulfate de sodium anhydre et évapore à sec. Les cristaux obtenus sont recristallisés dans l'acétate d'éthyle ou l'éther diisopropylique.

Cristaux blancs, rendement: 88%, F = 147°C.

Exemple 2

*[Nα-terbutyloxycarbonyl) p-cyanophénylalanyl]-1 pipéridine*

$$(XII: R' = -\underset{\underset{O}{\|}}{C}-OC(CH_3)_3;$$

$NR_3R_4$ = pipéridino). Activation de la fonction acide du composé

$$(IX: R' = -\underset{\underset{O}{\|}}{C}-OC(CH_3)_3)$$

(exemple 1) en anhydride carbonique mixte

$$(X: A = -\underset{\underset{O}{\|}}{C}-O-\underset{\underset{O}{\|}}{C}-OC_2H_5)$$

On ajoute à 0°C, sous atmosphère inerte, 4,2 g (0,0416 mole) de triéthylamine à une suspension de 11 g (0,0378 mole) de Nα-(terbutyloxycarbonyl) p-cyanophénylalanine (exemple 1) dans 125 ml de dichlorométhane. Au mélange devenu homogène, on ajoute, goutte à goutte, une solution de 4,3 g (0,0395 mole) de chloroformiate d'éthyle dans 10 ml de dichlorométhane. Après la fin de l'addition, on abandonne 45 minutes le mélange réactionnel, à 0°C, puis ajoute, goutte à goutte, 3,4 g (0,0397 mole) de pipéridine dissoute dans 10 ml de dichlorométhane. On laisse revenir le milieu réactionnel à température ambiante et abandonne pendant 15 h à cette température. On extrait le milieu réactionnel avec une solution aqueuse saturée de bicarbonate de sodium. La phase organique, après décantation, est séchee sur sulfate de sodium anhydre et évaporée à sec. Le résidu huileux donne des cristaux blancs, après trituration avec de l'éther diisopropylique. Ces cristaux sont recristallisés dans l'éther diisopropylique.

Cristaux blancs, rendement: 81%, F = 132°C.

Exemple 3

*méthyl-4 [Nα-(terbutyloxycarbonyl) p-cyanophénylalanyl]-1 pipéridine*

$$(XII: R' = -\underset{\underset{O}{\|}}{C}-OC(CH_3)_3;\ NR_3R_4 = \text{méthyl-4 pipéridino}).$$

Activation par transformation de la fonction acide en une fonction ester activé, par utilisation du réactif de couplage non racémisant DCC/HOBT.

On dissout 2,78 g (0,01 mole) de Nα-(terbutyloxycarbonyl) p-cyanophénylalanine (exemple 1) dans 50 ml de dichlorométhane. Sous atmosphère inerte, à température ambiante, on ajoute successivement 1 g (0,01 mole) de méthyl-4 pipéridine, 1,35 g (0,01 mole) d'hydroxy-1 benzotriazole (HOBT), 1,1 g (0,01 mole) de triéthylamine. Au milieu réactionnel, on ajoute, à température ambiante, 2,06 g (0,01 mole) de N,N-dicyclohexylcarbodiimide (DDC) dissoute dans 80 ml de dichlorométhane et abandonne le milieu réactionnel pendant 15 h à température ambiante. On filtre le précipité de dicyclohexylurée que l'on élimine. Le filtrat organique est lavé avec une solution aqueuse staurée de bicarbonate de sodium, est séché sur sulfate de sodium anhydre. L'évaporation laisse un résidu que est trituré avec de l'éther diisopropylique. Les cristaux blancs obtenus sont recristallisés dans l'acétate d'éthyle.

Cristaux blancs, rendement: 84%, F = 142°C (acétate d'éthyle)

Exemple 4

*Benzyl-4 [Nα-(terbutyloxycarbonyl) p-cyanophénylalanyl]-1 pipéridine*

$$(XII: R' = -\overset{\overset{O}{\|}}{C}-OC(CH_3)_3;\ NR_3R_4 = \text{benzyl-4 pipéridino}).$$

Activation par transformation de la fonction acide en une fonction ester activé, par utilisation du réactif de couplage non racémisant BOP.

On dissout 2,78 g (0,01 mole) de Nα-(terbutyloxycarbonyl) p-cyanophénylalanine (exemple 1) dans 100 ml d'acétonitrile et à température ambiante, sous atmosphère d'argon, on ajoute successivement 4,43 g (0,01 mole) d'hexafluorophosphate de benzotriazolyl-1 oxytris (diméthylamino) phosphonium (BOP), 1.75 g (0,01 mole) de benzyl-4 pipéridine et 1,1 g (0,01 mole) de triéthylamine. On abandonne, pendant 16 h , le milieu réactionnel à température ambiante. On filtre l'insoluble qui est écarté. Le filtrat est évaporé à sec et repris par du dichlorométhane. La phase organique est lavée avec une solution aqueuse saturée de

bicarbonate de sodium, puis séchée sur sulfate de sodium anhydre. L'évaporation à sec, laisse un résidu que l'on recristallise dans l'acétate d'éthyle.

Cristaux blancs, rendement: 78%, F = 131°C (acétate d'éthyle).

Exemple 5

*(Nα-méthyl p-cyanophénylalanyl)-1 pipéridine* (II: $R_2 = CH_3$; $NR_3R_4$ = pipéridino)

a) *Alkylation de la [Nα-(terbutyloxycarbonyl) p-cyanophénylalanyl]-1 pipéridine.*

A une suspension de 1,84 g (0,038 mole) d'hydrure de sodium, dipersé à 50% dans l'huile, dans 50 ml de diméthylformamide, on ajoute, goutte à goutte, à température ambiante, 13,1 g (0,0366 mole) de [Nα-(terbutyloxycarbonyl) p-cyanophénylalanyl]-1 pipéridine (exemple 2) dissoute dans 150 ml de diméthylformamide. On agite 45 minutes à température ambiante et ajoute, goutte à goutte, à cette température 6,24 g (0,044 mole) d'iodure de méthyle. A la fin de l'addition, on abandonne une nuit à température ambiante. On évapore à sec le diméthylformamide et reprend le résidu avec de l'eau. On extrait la phase aqueuse à l'éther. Les extraits éthérés, après séchage sur sulfate de sodium anhydre et évaporation du solvant, laissent un résidu huileux, constitué de [(Nα-méthyl Nα-(terbutyloxycarbonyl)) p-cyanophénylalanyl]-1 pipéridine

(XIII: $R_1 = CH_3$; $R = -\overset{\overset{O}{\|}}{C}-OC(CH_3)_3$; $NR_2R_3$ = pipéridino)

qui est utilisé dans l'étape subséquente b) sans autre purification.

b) *Déprotection*

Le résidu huileux obtenu dans l'étape précédente est dissous dans 150 ml d'une solution saturée de gaz chlorhydrique dans l'acétate d'éthyle et abandonné pendant 2 hr à température ambiante, puis à 0°C pendant une nuit. Les cristaux obtenus sont filtés, lavés à l'éther et recristallisés dans l'isopropanol.

Cristaux blancs, rendement: 70%, F = 234°C (isopropanol), chlorhydrate. (rendement global des étapes a) + b) = 70%)

Exemple 6

*(Nα-éthyl p-cyanophénylalanyl)-1 pipéridine* (II: $R_2 = C_2H_5$; $NR_3R_4$ = pipéridino)

Préparée selon le mode opératoire décrit dans l'exemple 5, par alkylation avec l'iodure d'éthyle de la [Nα-(terbutyloxycarbonyl) p-cyanophénylalanyl]-1 pipéridine (exemple 2), suivie de la déprotectioon par une solution saturée de gaz chlorhydrique dans l'acétate d'éthyle.

Cristaux blancs, rendement global: 69%, F= 226°C (isopropanol), chlorhydrate.

Exemple 7

*Méthyl-4 (Nα-méthyl p-cyanophénylalanyl)-1 pipéridine* (II: $R_2 = CH_3$; $NR_3R_4$ = méthyl-4 pipéridino).

Préparée selon le mode opératoire décrit dans l'exemple 5, par alkylation avec l'iodure de méthyle de la méthyl-4 [Nα-(terbutyloxycarbonyl) p-cyanophénylalanyl]-1 pipéridine (exemple 3) suivie de la déprotection par une solution saturée de gaz chlorhydrique dans l'acétate d'éthyle.

Cristaux blancs, rendement global: 80%, F = 214°C (isopropanol), chlorhydrate.

Exemple 8

*(Nα-éthyl p-cyanophénylalanyl)-1 méthyl-4 pipéridine* (II: $R_2 = C_2H_5$; $NR_3R_4$ = méthyl-4 pipéridino).

Préparée selon le mode opératoire décrit dans l'exemple 5, par alkylation avec l'iodure d'éthyle de la méthyl-4 [Nα-(terbutyloxycarbonyl) p-cyanophénylalanyl]-1 pipéridine (exemple 3), suivie de la déprotection par une solution saturée de gaz chlorhydrique dans l'acétate d'éthyle.

Cristaux blancs, rendement global: 77%, F = 220°C (isopropanol), chlorhydrate.

Exemple 9

*Benzyl-4 (Nα-méthyl p-cyanophénylalanyl)-1 pipéridine* (II: $R_2 = CH_3$; $NR_3R_4$ = benzyl-4 pipéridino).

Préparée selon le mode opératoire décrit dans l'exemple 5, par alkylation avec l'iodure de méthyle de la benzyl-4 [Nα-(terbutyloxycarbonyl) p-cyanophénylalanyl]-1 pipéridine (exemple 4), suivie de la déprotection par une solution saturée de gaz chlorhydrique dans l'acétate d'éthyle.

Cristaux blancs, rendement global: 72%, F = 202°C (isopropanol), chlorhydrate.

Exemple 10

*[Nα-(n-butyl) p-cyanophénylalanyl]-1 pipéridine* (I: $R_2 = n-C_4H_9$; $NR_3R_4$ = pipéridino).

a) *alkylation*

A 2,8 g ((0,0587 mole) d'hydrure de sodium à 50% dans l'huile, en suspension dans 100 ml de diméthylformamide, on ajoute, goutte à goutte, 20 g (0,0558 mole) de [Nα-(terbutyloxycarbonyl) p-cyano-

phénylalanyl]-1 pipéridine (exemple 2) dissoute dans 100 ml de diméthylformamide. Après 45 minutes à température ambiante, sous atmosphère d'argon, on ajoute 9,2 g (0,067 mole) de bromure de n-butyle et 10 g (0,067 mole) d'iodure de sodium. On abandonne la nuit à température ambiante. On verse le milieu réactionnel dans l'eau et évapore à sec. Le résidu est repris par de l'eau et la solution aqueuse est extraite à l'éther. Les extraits éthérés, séchés sur sulfate de sodium anhydre, laissent, après évaporation un résidu huileux, constitué de [Nα(n-butyl) Nα-(terbutyloxycarbonyl) p-cyanophénylalanyl]-1 pipéridine

$$(XIII: R' = -\overset{\overset{\displaystyle O}{\|}}{C}-OC(CH_3)_3;\ R_2 = \text{n-butyle};\ NR_3R_4 = \text{pipéridino})$$

que l'on utilise ans autre purification dans l'étape de déprotection ultérieure.

b) *Déprotection*

On utilise le procédé décrit dans l'exemple 5b).

Cristaux blancs, rendement global: 78%, F = 216°C (isopropanol), chlorhydrate.

Exemple 11

*(Nα-benzyl p-cyanophénylalanyl)-1 pipéridine* (II: $R_2 = CH_2-C_6H_5$; $NR_3R_4$ = pipéridino)

Préparée selon le mode opératoire décrit dans l'exemple 13, par alkylation avec le bromure de benzyle de la [Nα-(terbutyloxycarbonyl) p-cyanophényl-alanyl]-1 pipéridine (exemple 2) suivie de la déprotection par une solution saturée de gaz chlorhydrique dans l'acétate d'éthyle.

Cristaux blancs, rendement global: 64%, F = 212°C (isopropanol), chlorhydrate.

Exemple 12

*N-béta-naphtylsulfonyl-glycinate d'éthyle* (VIII: $A = C_2H_5$ $R_1$ = H; Ar = β-naphtyle).

A un mélange biphasique de 50 ml de solution aqueuse saturée de bicarbonate de sodium et de 50 ml de dichlorométhane, sous agitation mécanique rapide, à température ambiante, on ajoute par portions 10 g (0,072 mole) de glycinate d'éthyle (VII: $A = C_2H_5$; $R_1$ = H), puis 16,4 g (0,072 mole) de chlorure de β-naphtylsulfonyle. On abandonne le milieu réactionnel, sous bonne agitation, à température ambiante pendant 4 heures. On laisse décanter et écarte la phase aqueuse. La phase organique est récupérée et lavée avec une solution aquese d'acide chlorhydrique 2N. On sèche la phase organique sur du sulfate de sodium anhydre et évapore à sec. Par trituration du résidu huileux, obtenu après évaporation, avec de l'éther diisopropylique, on récupère des cristaux que l'on recristallise dans l'acétate d'éthyle.

Cristaux blancs, rendement: 91%, F = 80°C (acétate d'éthyle).

Exemple 13

*N-bétanaphtylsulfonyl-glycine* (III: $R_1$ = H; Ar = β-naphtyle)

A une solution de 18,8 g (0,064 mole) de N-bétanaphtylsulfonyl glycinate d'éthyle (exemple 12) dans 200 ml de méthanol, on ajoute 35 ml (0,07 mole) d'hydroxyde de sodium aqueux 2N et abandonne à température ambiante pendant 2 heures. On évapore le méthanol, reprend le résidu avec de l'eau. La phase aquese est extraite par de l'éther et les extraits éthérés écartés. Après neutralisation de la phase aqueuse avec 35 ml d'acide chlorhydrique 2N, on filtre les cristaux obtenus, les lave à l'eau et les sèche.

Cristaux blancs, rendement: 73% F = 157°C.

Les exemples 14 à 20 ont été réalisés selon le même mode opératoire que celui décrit dans l'exemple 12. Ils conduisent aux composés de formule générale (VIII: Ar = β-naphtyle; $A = CH_3$) et résultent de la N-sulfonylation des esters méthyliques des amino-acides (VII: A = $CH_3$) de configuration "R" ou "S", par le chlorure de β-naphtylsulfonyle. Ils sont regroupés dans le tableau suivant:

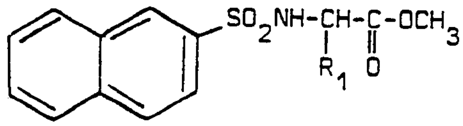

(VIII : Ar = β-naphtyle ; A = $CH_3$)

| Exemple | $R_1$ | Configuration de l'aminoacide | Rendement | F°(isopropanol) |
|---|---|---|---|---|
| 14 | $CH_3$ | R | 72 % | 98° C |
| 15 | $CH_3$ | S | 64 % | 97° C |
| 16 | $CH(CH_3)_2$ | S | 67 % | 106° C |
| 17 | $CH_2OH$ | S | 40 % | 150° C |
| 18 | $CH_2-C_6H_5$ | R | 64 % | 160° C |
| 19 | $C_6H_5$ | R | 84 % | 158° C |
| 20 | $CH(CH_3)OH$ | S | 50 % | 142°C |

Les exemples 21 à 27 ont été réalisés selon le même mode opératoire que celui décrit dans l'exemple 13. Ils conduisent aux acides de formule générale (III: Ar = β-naphtyle) et résultent de la saponification des esters de formule générale (VIII: Ar = β-naphtyle, A = $CH_3$). Ils sont regroupés dans le tableau suivant:

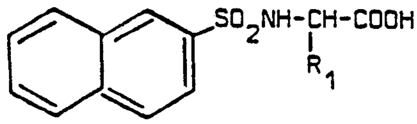

(III : Ar = β-napthyle)

| Exemple | $R_1$ | Configuration de l'aminoacide | Rendement | F° C |
|---|---|---|---|---|
| 21 | $CH_3$ | R | 89 % | 116° C |
| 22 | $CH_3$ | S | 90 % | 124° C |
| 23 | $CH(CH_3)_2$ | S | 76 % | 166° C |
| 24 | $CH_2OH$ | S | 76 % | 210° C |
| 25 | $CH_2-C_6H_5$ | R | 90 % | 60° C |
| 26 | $C_6H_5$ | R | 93 % | 160° C |
| 27 | $CH(CH_3)OH$ | R | 66 % | 194° C |

Exemple 28

*N-(quinoléinyl-8 sulfonyl)-glycinate d'éthyle* (VIII: Ar = quinoléinyle; A = $C_2H_5$; $R_1$ = H)

Préparé selon le mode opératoire décrit dans l'exemple 12, par sulfonylation du glycinate d'éthyle (VII: A = $C_2H_5$; $R_1$ = H) par le chlorure de (quinoléinyl-8) sulfonyle.

Cristaux blancs, F = 112°C (acétate d'éthyle); rendement: 91%.

Exemple 29

*N-(quinoléinyl-8 sulfonyl)-glycine* (III: Ar = quinoléinyle-8; $R_1$ = H)

Préparé selon le mode opératoire décrit dans l'exemple 13.

Cristaux blancs, rendement: 99%; F = 129°C.

Exemple 30

*[Nα-méthyl Nα-(N'-bétanaphytylsulfonylglycyl) p-cyanophénylalanyl]-pipéridine* (V: Ar = β-naphtyle; $R_1$ = H; $R_2$ = $C_2H_5$; $NR_3R_4$ = pipéridino)

On porte au reflux, pendant une heure, sous atmosphère inerte, 2,1 g (0,078 mole) de N(bétanaphtylsulfonyl)-glycine (exemple 2) dans 20 ml de chlorure de thionyle. On évapore à sec le milieu réactionnel et dissout le résidu huileux dans 50 ml de dichlorométhane. Le chlorure d'acide que l'on a dissous dans le dichlorométhane est ajouté goutte à goutte sous atmosphère inerte, à une solution de 1 g (0,0031 mole) de chlorhydrate de (Nα-méthyl p-cyanophénylalanyl)-1 pipéridine (II: $R_2$ = $CH_3$; $NR_3R_4$ = pipéridino) et de 1,14 g (0,0112 mole) de triéthylamine, dans 20 ml de dichlorométhane, qui a préalablement été refroidie entre 0°C et 5°C. On abandonne le milieu réactionnel à température ambiant pendant 20 heures. Les sels insolubles sont filtrés et le filtrat est évaporé à sec. On reprend le résidu par de l'acide chlorhydrique 1N et extrait la phase aqueuse acide obtenue par du dichlorométhane. Les extraits organiques sont séchés sur sulfate de sodium anhydre et évaporés à sec. Le résidu obtenu après évaporation est purifié par chromatographie sur une colonne de silice (élution toluène-acétate d'éthyle 1:1). On obtient des cristaux blancs.

Cristaux blancs, rendement: 69%; F = 130°C (isopropanol)

*Les exemples 31 à 33* ont été réalisés selon le même mode opératoire que celui décrit dans l'exemple 30. Ils conduisent aux nitriles de formule (V: Ar = β-naphtyle, $R_1$ = H) et résultent du couplage des synthons de formule générale (II) avec les acides de formule (III: Ar = β-napthyle; $R_1$ = H), préalablement activés en

halogénure d'acide, par traitement avec le chlorure de thionyle de préférence. Ils sont regroupés dans le tableau suivant:

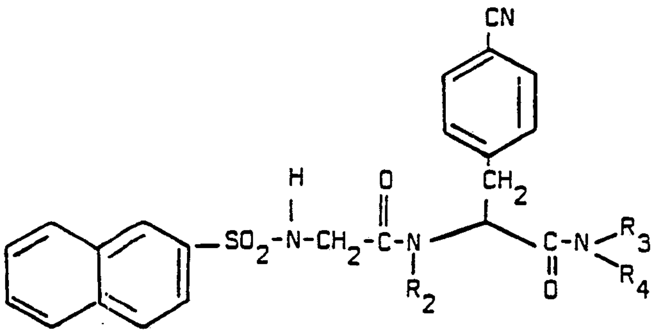

| Exemple | $R_2$ | $NR_3R_4$ | Rendement | F° C solvant recristallisation |
|---|---|---|---|---|
| 31 | $CH_2-C_6H_5$ | 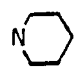 | 44 % | huile |
| 32 | $C_2H_5$ | 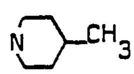 | 49 % | 74° C (éther diisopropylique) |
| 33 | $n-C_4H_9$ | 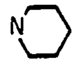 | 40 % | 142° C (isopropanol) |

Exemple 34

*[Nα-éthyl Nα-(N'-bétanaphtylsulfonylglycyl) p-cyanophénylalanyl]-1 pipéridine* (V: Ar = β-naphtyle; $R_1$ = H; $R_2$ = $C_2H_5$; $NR_3R_4$ = pipéridino)

A une suspension de 6,1 g (0,023 mole) de N(bétanaphtylsulfonyl)glycine (exemple 13) dans 80 ml de dichlorométhane, maintenue entre 0°C et 5°C, on ajoute 2,6 g (0,0253 mole) de triéthylamine, puis goutte à goutte 3,4 g (0,025 mole) de chloroformate d'isobutyle et on abandonne 1 heure à cette température. On ajoute alors 7,5 g (0,024 mole) de (Nα-éthyl p-cyanophénylalanyl)-1 pipéridine (II: $R_2$ = $C_2H_5$; $NR_3R_4$ = pipéridino), dissoute dans 50 ml de dichlorométhane et on abandonne à température ambiante, le milieu réactionnel pendant 20 heures. On évapore à sec, reprend le résidu par de l'eau. La phase aqueuse est extraite par du dichlorométhane. Les extraits organiques sont séchés sur sulfate de sodium anhydre et évaporés à sec. Le résidu huileux est purifié par chromatographie sur colonne de silice (élution toluène-acétate d'éthyle 1:1).

Cristaux blancs, rendement: 74,5% F = 82°C (acétate d'éthyle).

Exemple 35

*[Nα-méthyl Nα(N'-bétanaphtylsulfonyl-(S)-alanyl) p-cyanophénylalanyl]-1 pipéridine* (V: Ar = β-naphtyle; $R_1$ = $CH_3$; $R_2$ = $CH_3$; $NR_3R_4$ = pipéridino).

a) *Couplage n-induisant pas de racémisation utilisant le réactif de couplage hydroxy-1 benzotriazole (HOBT)/N,N-dicyclohexylcarbodiimide (DCC).*

A une suspension de 13 g (0,0425 mole) de chlorhydrate de (Nα-méthyl p-cyanophénylalanyl)-1 pipéridine (II: $R_2$ = $CH_3$; $NR_3R_4$ = pipéridino) dans 200 ml de dichlorométhane on ajoute successivement

15,1 g (0,0425 mole) de Nα-(bétanaphtylsulfonyl)-(S)-alanine (III = exemple 22, 4,3 g (0,0425 mole) de triéthylamine, 6,5 g (0,0425 mole) d'hydroxy-1 benzotriazole (HOBT). On refroidit le milieu réactionnel entre 0°C et +5°C et ajoute goutte à goutte 8,8 g (0,0425 mole) de N,N-dicyclohexylcarbodiimide (DCC) dissoute dans 50 ml de dichlorométhane. On abandonne le milieu réactionnel, sous bonne agitation, à température ambiante, pendant 17 heures. On filtre le précipité de dicyclohexylurée et le filtrat organique est lavé avec une solution aqueuse saturée de bicarbonate de sodium. La phase organique, séchée sur sulfate de sodium anhydre est évaporée à sec. L'évaportion du solvant laisse un résidu qui est trituré avec de l'acétate d'éthyle. Les cristaux blancs sont filtrés et lavés avec l'ether diisopropylique.

Cristaux blancs, rendement: 62%, F = 110°C (acétate d'éthyle).

b) *Couplage n'induisant pas de racémisation, utilisant le réactif de couplage hexafluorophosphate de benzotriazolyl-1 oxytris (diméthylamino) phosphonium (BOP).*

A une solution de 7,5 g (0,027 mole) de Nα-(bétanaphtylsulfonyl-(S)-alanine (III: exemple 22) dans 300 ml d'acétonitrile, on ajoute successivement 11,9 g (0,027 mole) d'hexafluorophosphate de benzotriazolyl-1 oxytris (diméthylamino) phosphonium (BOP), 8,3 g (0,027 mole) de chlorhydrate de (Nα-méthyl p-cyanophénylalanyl)-1 pipéridine (II: $R_2$ = $CH_3$; $NR_3R_4$ = pipéridino), 5,5 g (0,054 mole) de triéthylamine. On abandonne le milieu réactionnel sous atmosphère inerte, sous bonne agitation, à température ambiante, pendant 20 heures. On dilue le milieu réactionnel avec de l'acétate d'éthyle et le lave successivement avec une solution aqueuse saturée de chlorure de sodium, une solution d'acide chlorhyrique 2N, de l'eau, une solution aqueuse saturée de bicarbonate de sodium, puis de l'eau. La phase organique est séchée sur sulfate de sodium anhydre et évaporée à sec.

Le résidu est purifié par chromatographie sur une colonne de silice (élution: toluène-acétate d'éthyle 1:1). On récupère des cristaux blancs que l'on sèche.

Cristaux blancs, rendement: 42%, F = 110°C.

*Les exemples 36 à 41* sont effectués selon le même mode opératoire que celui décrit dans l'exemple 35a. Ils conduisent aux nitriles de formule (V) et résultent du couplage des synthons de formule générale (II) avec les acides de formule générale (III) préalablement activés par la transformation de la fonction acide en

fonction ester activé, utilisant le réactif de couplage non racémisant DCC/HOBT. Ils sont regroupés dans le tableau suivant:

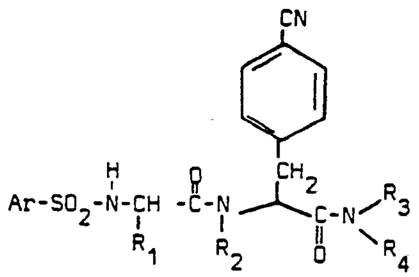

| Exemple | Ar | $R_1$ | $R_2$ | $NR_3R_4$ | Rendement | F° C |
|---|---|---|---|---|---|---|
| 36 | 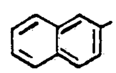 | H | $CH_3$ | 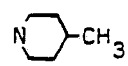 | 92 % | 143° C |
| 37 | 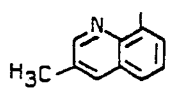 | H | $CH_3$ | 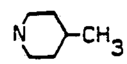 | 37 % | 202° C |
| 38 | 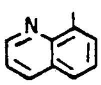 | H | $CH_3$ | 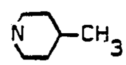 | 51 % | 102° C |
| 39 | 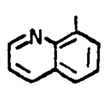 | H | $CH_3$ | 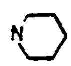 | 56 % | 60° C |
| 40 | 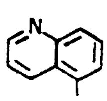 | H | $CH_3$ | 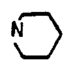 | 35 % | 212° C |
| 41 | 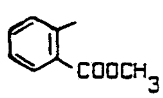 | H | $CH_3$ | 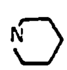 | 88 % | 100° C (pâteux) |

*Les exemples 42 à 45* utilisent le même mode opératoire que celui décrit dans l'exemple 35b. Ils conduisent aux nitriles (V: Ar = β-naphtyle) et résultent du couplage des synthons de formule générale (II) avec les acides de formule générale (III), préalablement activés par transformation de la fonction acide en

fonction ester activé, utilisant le réactif de couplage non racémisant BOP.

Ils sont regroupés dans le tableau suivant:

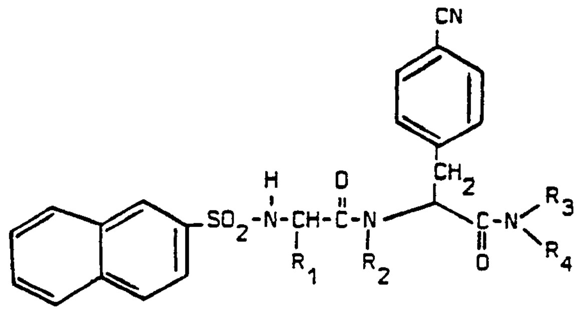

| Exemple | $R_1$ (configuration de l'aminoacide) | $R_2$ | $NR_3R_4$ | Rendement | F° C |
|---|---|---|---|---|---|
| 42 | H | $CH_3$ | 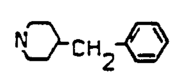 | 40 % | 82° C |
| 43 | $CH(CH_3)_2$ (S). | $CH_3$ | 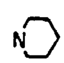 | 40 % | 228° C |
| 44 | $CH_2OH$(S) | $CH_3$ | 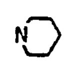 | 67 % | 102° C |
| 45 | $CH(CH_3)OH$(S) | $CH_3$ | 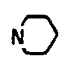 | 66 % | 98° C |

Exemple 46

*[Nα-méthyl Nα-(N'-bétanaphtylsulfonylglycyl) p-amidinophénylalanyl)]-1 pipéridine* (I: Ar = β-napthtyle; $R_1$ = H; $R_2$ = $CH_3$; $NR_3R_4$ = pipéridino).

dérivé n°1

a) *Formation de l'imidoester*

On sature à 0°C, sous atmosphère inerte, 80 ml de méthanol avec du gaz chlorhydrique et on ajoute, en une seule fois, à cette solution 5,3 g (0,01 mole) de [Nα-méthyl Nα-(N'-bétanaphtylsulfonylglycyl) p-cyanophénylalanyl]-1 pipéridine (V: exemple 30 et on abandonne 20 heures à 0°C. On évapore à sec, sans chauffer, le méthanol et obtient une résine blanche, constituée du chlorydrate de l'imidoester de formule générale (VI: Ar = β-naphtyle; $R_1$ = H; $R_2$ = $CH_3$; $NR_3R_4$ = pipéridino; X = $CH_3$) qui est utilisé sans autre purification dans l'étape ultérieure.

b) *Formation de l'amidine*

On sature à 0°C—5°C, sous atmosphère inerte, 80 ml de méthanol, avec de l'ammoniac gazeux, et ajoute à cette solution méthanolique ammoniacale, la résine blanche, obtenue dans l'étape précédente (exemple 46a), après dissolution dans 20 ml de méthanol. On porte au reflux le mélange réactionnel, sous atmosphère inerte, pendant 3 heures. On évapore à sec et reprend le résidu par de l'acide chlorhydrique 1N, en excès. La phase aqueuse acide est extraite par du dichlorométhane. La phase organique est séchée sur du sulfate de sodium anhydre et évaporée à sec. Le résidu semi-cristallin obtenu est dissous dans l'eau. La solution aqueuse obtenue est extraite par l'acétate d'éthyle, et les extraits organiques sont isolés. La phase organique est lyophilisée et le résidu semi-cristallin est trituré par de l'éther éthylique. Les cristaux blancs sont filtrés, lavés à l'éther et séchés. Le produit final est sous forme de chlorhydrate hydraté.

Cristaux blancs, rendement: 70%, F = 170°C (chlorhydrate, dihydrate).

Pour les dérivés 2 à 15, on utilise les mêmes modes opératoires que ceux décrits dans l'exemple 46. Ils conduisent aux Nα-arylsulfonylaminoacyl p-amidinophénylalaninamides de formule générale (I) et

résultent de la transformation des nitriles de formule générale (V) en amidines de formule générale (I) par l'intermédiaire des imidoesters de formule générale (VI). Ils sont regroupés dans le tableau suivant:

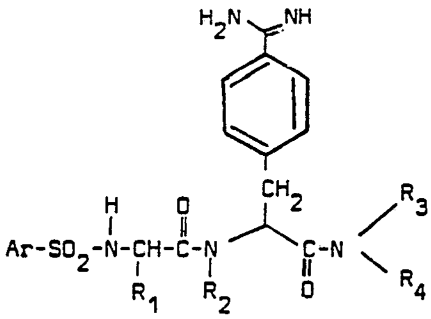

, HCl, $xH_2O$

| Dérivé | Ar | $R_1$ (configuration de l'aminoacide) | $R_2$ | $NR_3R_4$ | x | Rendement | F° C |
|---|---|---|---|---|---|---|---|
| 2 | 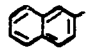 | H | $CH_3$ | 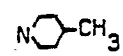 | 1,5 | 64 % | 160° |
| 3 | 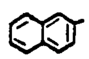 | H | $C_2H_5$ | 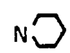 | 1,5 | 56 % | 168° |
| 4 | 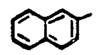 | H | $CH_2$- 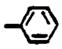 | 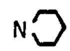 | 2 | 64 % | 170° |
| 5 | 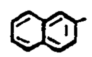 | H | $C_2H_5$ | 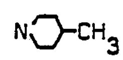 | 1 | 50 % | 164° |
| 6 | 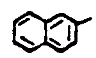 | H | n-$C_4H_9$ | 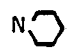 | 1,5 | 54 % | 154° |
| 7 | 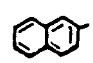 | $CH_3$ (S) | $CH_3$ | 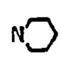 | 1,5 | 71 % | 162° |
| 8 | 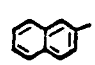 | H | $CH_3$ | 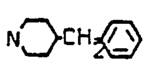 | 1,5 | 57 % | 160° |
| 9 | 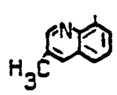 | H | $CH_3$ | 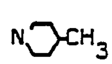 | 2 | 46 % | 196° |
| 10 | 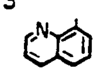 | H | $CH_3$ | 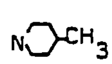 | 3 | 76 % | 165° |
| 11 | 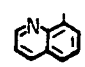 | H | $CH_3$ | 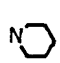 | 4,5 | 51 % | 160° |
| 12 | 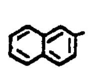 | $CH(CH_3)_2$ (S) | $CH_3$ | 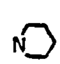 | 3 | 48 % | 175° |
| 13 | 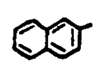 | $CH(CH_3)OH$ (S) | $CH_3$ | 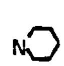 | 2 | 76 % | 169° |
| 14 | 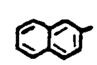 | $CH_2OH$ (S) | $CH_3$ | 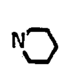 | 1,5 | 72 % | 170° |
| 15 | 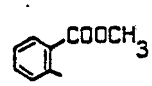 | H | $CH_3$ | 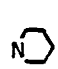 | 1,5 | 28 % | 158° |

Les résultats des études toxicologique et pharmacologique qui sont rapportées ci-dessus ont mis en évidence les intéressantes propriétés des composés de l'invention.

Ces derniers sont doués d'une très bonne activité inhibitrice de la thrombine et possèdent en outre de remarquables propriétés antithrombotiques in vivo que ne manifestent pas les composés des formules (A) et (B) et le composé (C).

Comparés à l'héparine, ils ont une durée d'action bien supérieure sans induire d'augmentation du temps de saignement.

L'invention a donc encore pour objet un médicament présentant en particulier des propriétés antithrombotiques, caractérisé en ce qu'il contient, à titre de principe actif, un composé de formule (I) ou un sel d'addition avec un acide minéral ou organique thérapeutiquement acceptable.

Etude toxicologique:

Les composés de l'invention bénéficient d'une bonne tolérance et d'une faible toxicité. Les essais effectués sur différentes espèces animales les toxicités aigue, subchronique, chronique n'ont pas mis en évidence une quelconque réaction locale ou générale, perturbation ou anomalie dans les examens biochimiques, macroscopiques et microscopiques effectués tout au long des essais.

Etude pharmacologique:

Dans cette étude, les composés de l'invention ont été comparés à l'héparine et à la [Nα-(N-bétanaphtylsulfonylglycyl) p-amidinophénylalanyl]-1 pipéridine, composé de structure proche décrit comme un puissant inhibiteur de la thrombine (J. HAUPTMANN et al. Thromb, Res, *39*, 771—775, 1983) et qui sera nomme' dérivé C.

1) Détermination de la spécifité vis-à-vis de la thrombine.

De nombreuses sérine-protéases (facteurs XIIa, IXa, VIIa, Xa, plasmine thrombine) existent dans le plasma et interviennent dans le mécanisme de la coagulation. Afin de ne pas induire des perturbations trop importantes dans la "cascade de la coagulation" et entraîner des risques hémorragiques, il convient de s'assurer que les composés choisis possèdent une action spécifique sur la thrombine. D'autre part, afin d'obtenir une bonne activité par la voie orale, il est nécessaire aussi d'avoir une bonne spécificité vis-à-vis de la trypsine, sérine-protéase du tractus digestif.

On a ainsi déterminé selon la méthode de Dixon (Biochem. J., 1953, *55*, 170-171) les constantes d'inhibition de la thrombine bovine (Sigma 2000 MH/mg) in vitro sur l'hydrolyse du substrat 2238 (Kabi Vitrum) à pH 8 et à 25°C et celles de la trypsine bovine (Sigma type III-S) dans les mêmes conditions.

Les résultats sont rassemblés dans le tableau suivant:

| Dérivé | $K_1$ Thrombine | $K_1$ Trypsine |
|---|---|---|
| 1 | 0,27 $10^{-8}$M | 0,10 $10^{-6}$M |
| 11 | 1,52 $10^{-7}$M | 1,2 $10^{-6}$M |
| 2 | 2,15 $10^{-8}$M | 0,12 $10^{-6}$M |
| C | $10^{-8}$M | 0,75 $10^{-6}$M |

2) Temps de thrombine.

Le temps de coagulation du plasma citraté en présence de thrombine est mesuré ex vivo chez le rat selon la technique de BIGGS R. M. (Human blood coagulation, haemostasis and thrombosis; Oxford, Blackwell Scientific Publications, 1972)

Les prélèvements sont effectués une heure après administration souscutanée du composé à tester, par ponction à l'aorte abdominale. Le sang est receuilli sur citrate de sodium à 3,8% (1 volume pour 9 volumes de sang). Le plasma est obtenu par centrifugation à 2600 g pendant 10 minutes. A du plasma, on ajoute 0,2 ml d'une solution de thrombine (20 U/ml). Le temps de coagulation est enregistré.

Les résultats sont rassemblés dans le tableau suivant:

| | Dose mg/kg | Voie | Résultats (Temps en secondes) | % allongement | p |
|---|---|---|---|---|---|
| Témoin<br>Héparine | 10 | S.C<br>S.C | 6 ± 0<br>20 ± 4 | 233 | 0,001 |
| Témoin<br>Dérivé C | 10 | S.C<br>S.C | 7 ± 0<br>9 ± 0 | 29 | 0,001 |
| Témoin<br>Dérivé n°1 | 10 | S.C<br>S.C | 7 ± 0<br>114 ± 16 | 1529 | 0,001 |
| Témoin<br>Dérivé n°3 | 10 | S.C<br>S.C | 6 ± 0<br>18 ± 2 | 200 | 0,001 |
| Témoin<br>Dérivé n°11 | 10 | S.C<br>S.C | 8 ± 0<br>68 ± 6 | 750 | 0,001 |
| Témoin<br>Dérivé n°5 | 10 | S.C<br>S.C | 9 ± 0<br>12 ± 1 | 33 | 0,001 |
| Témoin<br>Dérivé n°13 | 10 | S.C<br>S.C | 8 ± 0<br>28 ± 5 | 255 | 0,05 |
| Témoin<br>Dérivé n°14 | 10 | S.C<br>S.C | 7 ± 0<br>105 ± 17 | 1370 | 0,01 |

3) Thrombose veineuse à la vrille.

Les essais ont été effectués selon une adaptation de la méthode de T. KUMADA et al. (Thromb. Res. *18*, 189—203, 1980).

Une spirale métallique (bourre-pâte de dentiste recoupée) est insérée dans la veine cave inférieure du rat anesthésié. Une heure auparavant, les animaux ont reçu par la voie sous-cutanée, le composé à tester. Cinq heures après, la spirale est retirée avec le thrombus qu'elle retient puis séchée par tamponnements répétés sur papier filtre et pesée. La spirale est ensuite débarrassée du thrombus, séchée et pesée à nouveau. La différence, pondérale donne le poids du thrombus.

Les résultats sont rassemblés dans le tableau suivant:

| Produit | Dose mg/kg | Poids du thrombus en mg | Variation | p |
|---|---|---|---|---|
| Témoin | | 4,47 ± 0,51 | | |
| Héparine | 5 | 2,91 ± 0,53 | -35 % | 0,05 |
| Héparine | 10 | 1,62 ± 0,34 | -64 % | 0,001 |
| Héparine | 20 | 0,26 ± 0,04 | -94 % | 0,001 |
| Témoin | | 4,77 ± 0,47 | | |
| Dérivé C | 20 | 3,99 ± 0,45 | -16 % | n.s. |
| Dérivé C | 50 | 3,63 ± 0,37 | -24 % | n.s. |
| Dérivé C | 100 | 3,08 ± 0,28 | -35 % | 0,01 |
| Témoin | | 3,51 ± 0,53 | | |
| Dérivé n°1 | 5 | 2,43 ± 0,17 | -31 % | n.s. |
| Dérivé n°1 | 10 | 2,01 ± 0,20 | -43 % | 0,05 |
| Dérivé n°1 | 20 | 1,34 ± 0,12 | -62 % | 0,01 |
| Dérivé n°1 | 50 | 0,87 ± 0,07 | -75 % | 0,001 |
| Témoin | | 4,53 ± 0,55 | | |
| Dérivé n°3 | 10 | 2,46 ± 0,16 | -46 % | 0,01 |
| Témoin | | 3,85 ± 0,21 | | |
| Dérivé n°13 | 10 | 1,36 ± 0,14 | -65 % | 0,001 |

| Produit | Dose mg/kg | Poids du thrombus en mg | Variation | p |
|---|---|---|---|---|
| Témoin | | 4,10 ± 0,43 | | |
| Dérivé n°11 | 5 | 3,13 ± 0,38 | -24 % | n.s. |
| Dérivé n°11 | 10 | 2,02 ± 0,19 | -51 % | 0,001 |
| Dérivé n°11 | 20 | 1,85 ± 0,13 | -55 % | 0,001 |
| Témoin | | 4,11 ± 0,33 | | |
| Dérivé n°5 | 10 | 3,25 ± 0,24 | -21 % | 0,05 |
| Témoin | | 3,85 ± 0,21 | | |
| Dérivé n°14 | 10 | 0,79 ± 0,19 | -80 % | 0,001 |

Etude cinétique de la thrombose veineuse à la vrille.

Létude comparative de la cinétique d'effet portant sur l'héparine et le dérivé n° 1 a été effectuée.

Les dérivés à tester sont administrés par la voie sous-cutanée 15 mn, 1 H, 2 H, 4 H, 6 H, 16 H et 48 H avant la pose de la spirale qui est retirée 5 H après. On détermine le poids du thrombus.

Les résultats sont rassemblés dans le tableau ci-après.

| Produit | Dose mg/kg | Voie | Temps | Poids du thrombus en mg | Variation | p |
|---|---|---|---|---|---|---|
| Témoin | | s.c. | | 4,21 ± 0,34 | | |
| Héparine | 10 | s.c. | -15 mn | 4,21 ± 0,34 | -58 % | 0,001 |
| Héparine | 10 | s.c. | - 1 H | 0,82 ± 0,22 | -80 % | 0,001 |
| Héparine | 10 | s.c. | - 4 H | 1,18 ± 0,12 | -72 % | 0,001 |
| Héparine | 10 | s.c. | - 6 H | 2,67 ± 0,35 | -36 % | 0,001 |
| Témoin | | s.c. | | 4,55 ± 0,55 | | |
| Héparine | 10 | s.c. | - 2 H | 0,55 ± 0,09 | -88 % | 0,001 |
| Témoin | | s.c. | | 4,19 ± 0,37 | | |
| Héparine | 5 | s.c. | -15 mn | 3,70 ± 0,29 | -12 % | n.s. |
| Héparine | 5 | s.c. | - 1 H | 1,73 ± 0,26 | -59 % | 0,001 |
| Héparine | 5 | s.c. | - 4 H | 3,10 ± 0,43 | -26 % | n.s. |
| Héparine | 5 | s.c. | - 6 H | 3,15 ± 0,28 | -25 % | n.s. |
| Témoin | | s.c. | | 4,35 ± 0,43 | | |
| Héparine | 5 | s.c. | - 2 H | 3,04 ± 0,25 | -30 % | 0,05 |
| Témoin | | s.c. | | 3,37 ± 0,33 | | |
| Dérivé n°1 | 20 | s.c. | -15 mn | 1,99 ± 0,24 | -41 % | 0,01 |
| Dérivé n°1 | 20 | s.c. | - 1 H | 1,67 ± 0,15 | -50 % | 0,001 |
| Témoin | | s.c. | | 3,91 ± 0,42 | | |
| Dérivé n°1 | 20 | s.c. | - 2 H | 1,82 ± 0,10 | -53 % | 0,001 |
| Témoin | | s.c. | | 3,95 ± 0,32 | | |
| Dérivé n°1 | 20 | s.c. | - 4 H | 1,40 ± 0,13 | -63 % | 0,001 |
| Dérivé n°1 | 20 | s.c. | - 6 H | 1,40 ± 0,13 | -43 % | 0,001 |
| Témoin | | s.c. | | 3,52 ± 0,44 | | |
| Dérivé n°1 | 20 | s.c. | -16 H | 2,64 ± 0,38 | -25 % | n.s. |
| Témoin | | s.c. | | 3,20 ± 0,23 | | |
| Dérivé n°1 | 50 | s.c. | -15 mn | 1,31 ± 0,08 | -59 % | 0,001 |
| Dérivé n°1 | 50 | s.c. | - 1 H | 0,92 ± 0,08 | -71 % | 0,001 |
| Dérivé n°1 | 50 | s.c. | - 2 H | 1,01 ± 0,08 | -68 % | 0,001 |
| Dérivé n°1 | 50 | s.c. | - 4 H | 0,82 ± 0,07 | -74 % | 0,001 |
| Témoin | | s.c. | | 3,77 ± 0,36 | | |
| Dérivé n°1 | 50 | s.c. | - 6 H | 1,21 ± 0,16 | -68 % | 0,001 |
| Témoin | | s.c. | | 3,70 ± 0,24 | | |
| Dérivé n°1 | 50 | s.c. | -16 H | 1,69 ± 0,26 | -54 % | 0,001 |
| Témoin | | s.c. | | 2,98 ± 0,39 | | |
| Dérivé n°1 | 50 | s.c. | -48 H | 2,49 ± 0,30 | -16 % | n.s. |

4) Temps de saignement.

Cette étude a été effectuée selon une daptation de la technique de L. STELLA et al. (Thromb. Res.; 1975, 7, 709—716).

Après anethésie du rat au pentobarbital, la queue est sectionnée à 5 mm de l'extrémité et le sang de la blessure soigneusement tamponné toutes les 15 secondes à l'aide d'un papier filtre jusqu'à hémostase. Celle-ci est atteinte lorsqu'aucune tâche n'apparait pendant 1 minute. Les produits à tester sont administrés par la voie sous-cutanée, une heure avant la section de la queue.

Les résultats sont rassemblés dans le tableau suivant:

| Produit | Dose mg/kg | Durée en secondes | Extrêmes | p |
|---|---|---|---|---|
| Témoin | | 360 | 330-480 | |
| Héparine | 5 | 465 | 330-540 | n.s. |
| Héparine | 10 | 3600 | 525-> 3600 | 0,01 |
| Héparine | 20 | 3600 | 690-> 3600 | 0,01 |
| Témoin | | 540 | 405-675 | |
| Dérivé C | 10 | 375 | 360-510 | n.s. |
| Dérivé C | 20 | 750 | 420-960 | n.s. |
| Dérivé C | 50 | 600 | 435-615 | n.s. |
| Témoin | | 405 | 390-510 | |
| Dérivé n°1 | 10 | 450 | 420-525 | n.s. |
| Dérivé n°1 | 20 | 480 | 420-3600 | n.s. |
| Dérivé n°1 | 50 | 465 | 360->3600 | n.s. |
| Témoin | | 480 | 405-840 | |
| Dérivé n°1 | 100 | 795 | 600-1140 | n.s. |
| Dérivé n°1 | 100 | 960 | 600-1020 | 0,05 |
| Témoin | | 525 | 460-600 | |
| Dérivé n°11 | 5 | 615 | 540-780 | n.s. |
| Dérivé n°11 | 10 | 690 | 495-750 | n.s. |
| Dérivé n°11 | 20 | 585 | 495-855 | n.s. |
| Dérivé n°11 | 50 | 540 | 450-660 | n.s. |

Etude cinétique du temps de saignement.

L'étude comparative de la cinétique d'effet portant sur l'héparine et le dérivé n° 1 a été effectuée.

Les dérivés à tester sont administrés par la voie sous cutanée 15 mn, 1 H, 2 H, 4 H et 6 H avant l'anesthésie et la section de la queue.

Les résultats sont rassemblés dans le tableau suivant.

| Produit | Dose mg/kg | Temps | Durée en secondes | Extrêmes | p |
|---|---|---|---|---|---|
| Témoin | | | 330 | 240-405 | |
| Héparine | 10 | -15 mn | 465 | 315-510 | n.s. |
| Héparine | 10 | - 1 H | >3600 | 405->3600 | 0,05 |
| Héparine | 10 | - 4 H | >3600 | 600->3600 | 0,01 |
| Héparine | 10 | - 6 H | 390 | 315-495 | n.s. |
| Témoin | | | 412 | 360-540 | |
| Dérivé n°1 | 10 | -15 mn | 465 | 300-690 | n.s. |
| Dérivé n°1 | 10 | - 1 H | 555 | 390-570 | n.s. |
| Dérivé n°1 | 10 | - 4 H | 480 | 465-540 | n.s. |
| Dérivé n°1 | 10 | - 6 H | 450 | 390-870 | n.s. |
| Témoin | | | 465 | 360-795 | |
| Dérivé n°1 | 20 | -15 mn | 540 | 390-720 | n.s. |
| Dérivé n°1 | 20 | - 1 H | 480 | 435-630 | n.s. |
| Dérivé n°1 | 20 | - 2 H | 540 | 405-520 | n.s. |
| Dérivé n°1 | 20 | - 4 H | 585 | 405-1800 | n.s. |
| Dérivé n°1 | 20 | - 6 H | 450 | 300-840 | n.s. |

Ces études qui viennent d'être réalisées ont mis en évidence les effets remarquables des dérivés de l'invention.

pouvoir antithrombotique = la détermination du temps de thrombine et le test de la vrille ont montré que les composés de l'invention produisaient une activité bien supérieure au dérivé C et qu'ils possédaient sur l'héparine l'avantage d'une action beaucoup plus durable; en effet, si dans les premières heures, les effets de l'héparine et du dérivé n° 1 sont superposables, après 6 H, l'héparine accuse une baisse sensible alors que le dérivé n°1 produit encore une diminution du poids du thrombus de 68% et 48 H après de 16%.

On peut en conclure que, à activité antithrombotique équivalente, les dérivés de l'invention apportent une couverture remarquable dans le temps supérieure à 16 H par rapport à l'héparine (3—4 heures).

temps de saignement = l'étude cinétique a nettement montré le risque hémorragique induit par l'héparine. Le dérivé de l'invention allongeant très peu le temps de saignement, permet une marge de sécurité très supérieure à celle de l'héparine.

L'invention a encore pour objet un médicament présentant en particulier des activités antithrombotiques caractérisé en ce qu'il contient à titre de principe actif un dérivé de formule (I) ou un sel d'addition avec un acide minéral ou organique pharmaceutiquement acceptable.

Le médicament de l'invention peut être présenté pour l'administration orale sous forme de comprimés, comprimés dragéifiés, capsules, gouttes, sirop ou granulé.

Il peut aussi être présenté pour l'administration rectale sous forme de suppositoires et pour l'administration parentérale sous forme de soluté injectable.

Chaque dose unitaire contient avantageusement de 0,005 g à 0,500 g de principe actif en fonction de l'âge du malade et de la gravité de l'affection traitée. On donnera ci-après, à titre d'exemples non limitatifs, quelques formulations pharmaceutiques du médicament de l'invention.

1) Comprimés dragéifiés

Dérivé n° 1 0,050 g

Excipient Lactose, polyvinylpyrrolidone, stéréate de magnésium, gomme laque, talc, carbonate de calcium, silice, oxyde de titane, gomme arabique, cire blanche, cire de carnauba.

2) Comprimés

Dérivé n° 2 0,025 g

Excipient Lactose, cellulose microcristalline, talc, stéarate de magnésium.

3) Capsules
Dérivé n° 3 0,100 g
Excipient Talc, amidon de blé, stéarate de magnésium.

4) Suppositoires
Dérivé n° 5 0,050 g
Excipient Glycérides semi-synthétiques.

5) Soluté injectable
Dérivé n° 11 0,025 g
Excipient Solvant isotonique q.s.p. 3 ml

Pour ses propriétés anticoagulante et antithrombotique, dépourvu des effets secondaires dûs au risque hémorragique, le médicament de l'invention est utilement administré dans la prévention et le traitement de la maladie thrombo-embolique.

**Revendications pour les Etats contractants: BE CH DE FR GB IT LI LU NL SE**

1. Composés de formule

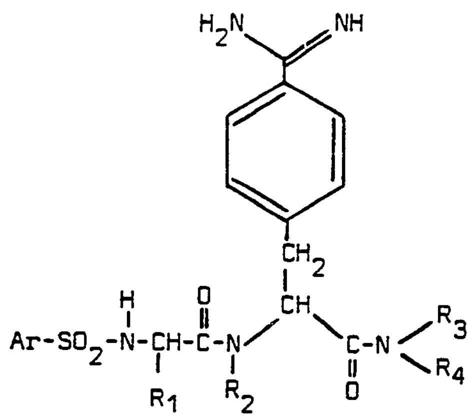

(I)

dans laquelle:

$R_1$ représente l'hydrogène, un groupe alcoyle ou hydroxyalcoyle en $C_1$—$C_6$, benzyle, un groupe phényle ou un groupe hydroxy-4 phényle;

$R_2$ représente un groupe alcoyle, alcényle ou alcynyle ayant jusqu'à 6 atomes de carbone, un groupe benzyle, un groupe alcoxy (en $C_1$—$C_6$) carbonylalcoyle en $C_1$—$C_6$, carboxyalcoyle en $C_1$—$C_6$, hydroxyalcoyle en $C_1$—$C_6$;

$R_3$ et $R_4$, identiques ou différents, représentent chacun un radical alcoyle, hydroxyalcoyle alcényle ou alcynyle ayant jusqu'à 6 atomes de carbone, ou forment ensemble, avec l'azote auquel ils sont rattachés, un hétérocycle sature choisi parmi morpholino, thiomorpholino, pyrrolidino non substitué ou substitué par un groupe alcoxy ($C_1$—$C_6$) carbonyle ou carboxy, pipérazino, (alcoyle en $C_1$—$C_6$)-4 pipérazino, (hydroxyalcoyle en $C_1$—$C_6$)-4 pipérazino, ou pipéridino non substitué ou substitué par un groupe alcoyle en $C_1$—$C_6$, benzyle, hydroxy, hydroxyalcoyle en $C_1$—$C_6$, amino, aminoalcoyle en $C_1$—$C_6$, hydroxyamino, alcoxy ($C_1$—$C_6$) carbonyle ou carboxy;

Ar représente un groupe phényle, alpha-naphtyle, béta-naphtyle, méthoxycarbonyl-phényle, ou bien un groupe hétéroaryle choisi parmi les radicaux pyridyle, quinoléinyle, isoquinoléinyle ou méthylquinoléinyle; et leurs sels d'addition avec les acides minéraux ou organiques pharmaceutiquement acceptables ainsi que les stéréoisomères ou leur mélange.

2. Composés selon la revendication 1, de formule (I) dans laquelle $R_1$ représente l'hydrogène ou un radical alcoyle.

3. Composés selon la revendication 1 ou 2, de formule (I) dans laquelle $R_2$ représente un radical alcoyle.

4. Composés selon la revendication 1, 2 ou 3, de formule (I) dans laquelle le groupe

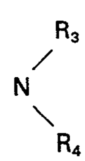

représente un radical pipéridino substitue par un groupe methyle ou benzyle ou non substitué.

5. Composés selon la revendication 1, 2, 3 ou 4, de formule (I) dans laquelle Ar représente un radical naphtyle.

6. [N α-méthyl Nα-(N-bétanaphtylsulfonylglycyl) p-amidinophénylalanyl]-1 pipéridine et ses sels pharmaceutiquement acceptables.

7. Méthyl-4 [Nα-méthyl Nα-(N-bétanaphtylsulfonylglycyl) p-amidinophénylalanyl]-1 pipéridine et ses sels pharmaceutiquement acceptables.

8. [Nα-éthyl Nα-(N-bétanaphtylsulfonylglycyl) p-amidinophénylalanyl]-1 pipéridine et ses sels pharmaceutiquement acceptables.

9. [Nα-éthyl Nα-(N-bétanaphtylsulfonylglycyl) p-amidinophénylalanyl]-1-méthyl-4 pipéridine et ses sels pharmaceutiquement acceptables.

10. [Nα-méthyl Nα-(N-quinoléinyl-8 sulfonylglycyl) p-amidinophénylalanyl]-1 pipéridine et ses sels pharmaceutiquement acceptables.

11. [Nα-méthyl Nα-(N-bétanaphtylsulonyl-(S)-séryl) p-amidinophénylalanyl]-1 pipéridine et ses sels pharmaceutiquement acceptables.

12. [Nα-méthyl Nα-(N-bétanaphtylsufonyl-(S)-thréonyl) p-amidinophénylalanyl]-1 pipéridine et ses sels pharmaceutiquement acceptables.

13. Procédé de préparation des composés selon les revendications 1 à 12 caractérisé en ce que l'on fait réagir sur la cyano-4 phénylalaninamide Nα-alcoylée de formule (II)

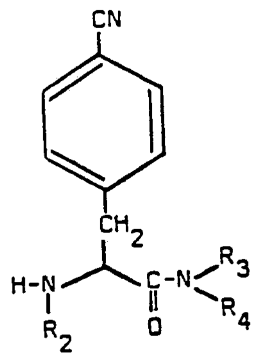

(II)

dans laquelle $R_2$, $R_3$ et $R_4$ ont les mêmes significations que dans la formule (I), une acide de formule

$$Ar—SO_2—NH—\underset{\displaystyle R_1}{\underset{|}{CH}}—COOH \qquad (III)$$

sous sa forme activée

$$Ar—SO_2—NH—\underset{\displaystyle R_1}{\underset{|}{CH}}—CO—R \qquad (IV)$$

dans laquelle Ar et $R_1$ ont les mêmes significations que dans la formule (I) et R représente un bon groupement nucléofuge, tel que chlore alcoxycarbonyloxy ou hétéroaryle, pour obtenir le composé de formule (V)

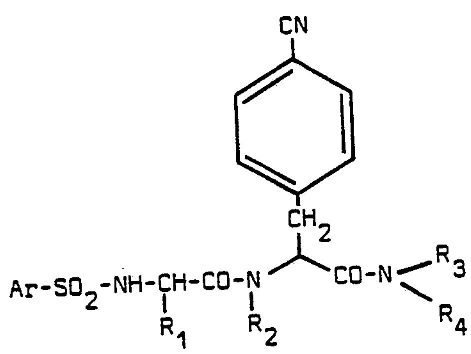

(V)

dans laquelle Ar, $R_1$, $R_2$, $R_3$ et $R_4$ ont les mêmes significations que dans la formule (I) qu'on traite avec un excès d'une solution saturée de gaz chlorhydrique dans un alcool de formule X—OH dans laquelle X

représente un radical alcoyle inférieur, pour obtenir l'imidoester de formule (VI) sous forme de chlorhydrate

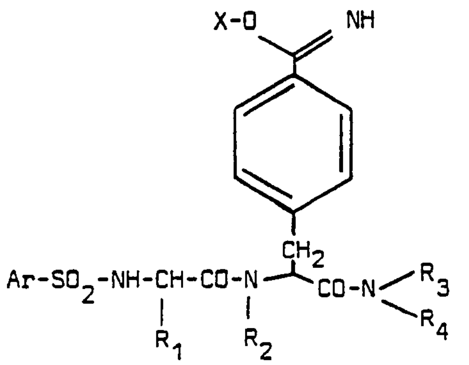

(VI)

dans laquelle Ar, $R_1$, $R_2$, $R_3$, $R_4$ et X ont les mêmes significations précitées qui est alors traité par un excès d'une solution de gaz ammoniac dans un alcool inférieur à la température d'ébullition du mélange réactionnel pour obtenir le composé de formule (I) recherché.

14. Procédé selon la revendication 13 caractérisé en ce que l'activation de l'acide de formule (III) est effectuée par action d'un chloroformate d'alcoyle.

15. Procédé selon la revendication 13 caractérisé en ce que l'activation de l'acide de formule (III) est effectuée par action de réactifs de couplage n'induisant pas la racémisation, tels que l'hydroxy-1 benzotriazole en présence de N,N dicyclohexylcarbodiimide, l'hexafluorophosphate de benzotriazolyl-1 oxytris (diméthylamino) phosphonium ou le chlorure de N,N-bis (oxo-2 oxazolidinyl-3) phosphorodiamidique.

16. Procédé selon l'une des revendications 13 à 15 caractérisé en ce que la préparation de l'imidoester de formule (VI) s'effectue en milieu alcoolique, en présence d'un acide fort.

17. Procédé selon l'une des revendications 13 à 16 caractérisé en ce que la formation de l'amidine de formule (I) s'effectue en milieu alcoolique, par action du gaz ammoniac.

18. Médicament caractérisé en ce qu'il contient, à titre de principe actif un dérivé de formule (I) suivant l'une des revendications 1 à 12 ou l'un de ses sels pharmaceutiquement acceptables.

19. Médicament selon la revendication 18, caractérisé en ce qu'il est présenté sous forme de doses unitaires contenant chacun de 0,005 g à 0,500 g de principe actif.

20. Compsés de formule (II)

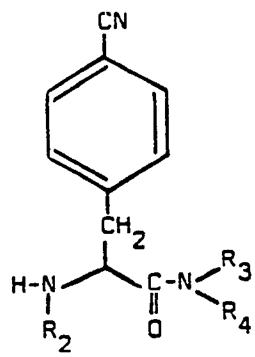

(II)

dans laquelle $R_2$, $R_3$ et $R_4$ ont la même signification que dans la revendication 1, ainsi que les isomères ou leurs mélanges et leurs sels d'acides minéraux ou organiques.

21. Procédé de préparation des composés de formule (II) selon la revendication 20, caractérisé en ce que l'on fait réagir l'acide de formule (IX) activée, par l'action de réactifs de couplage n'induisant pas la racémisation tels que l'hydroxy-1 benzotriazole en présence de N,N-dicyclohéxylcarbodiimide, l'hexafluorophosphate de benzatriazolyl-1 oxytris (diméthylamino) phosphonium ou le chlorure de N,N-bis (oxo-2 oxazolidinyl-3) phosphorodiamidique, en composé de formule (X)

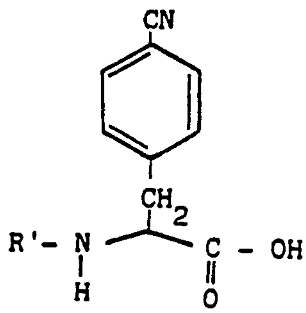

(IX)

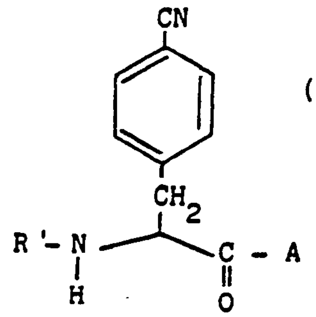

(X)

dans lesquelles R′ représente un groupement N-protecteur et A représente le reste du réactif de couplage, avec l'amine de formule

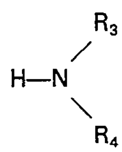

(XI)

dans laquelle $R_3$ et $R_4$ ont les mêmes significations que dans la formule II, pour former le composé de formule:

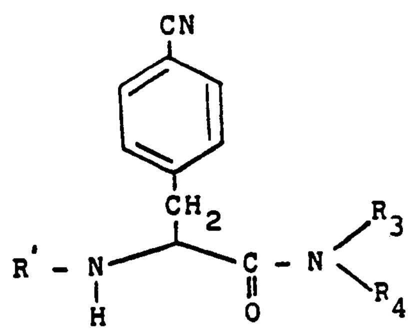

(XII)

dans laquelle R′, $R_2$ et $R_4$ ont les mêmes significations que dans les formules II et IX, que l'on fait réagir avec une composé $R_2$—X, dans lequel X est un halogène et $R_2$ est tel que dans formule II, pour obtenir le composé de formule

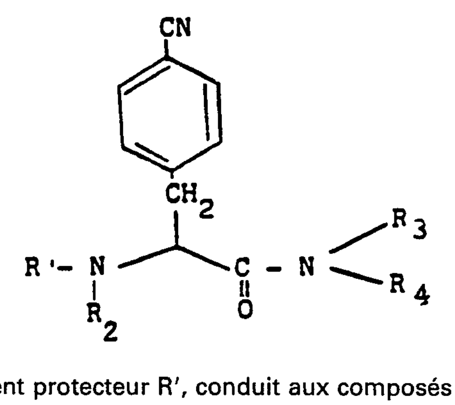

(XIII)

qui, par clivage du groupement protecteur R′, conduit aux composés de formule (II).

**Revendications pour les Etats contractants: AT GR ES**

1. Procédé de préparation de composés de formule:

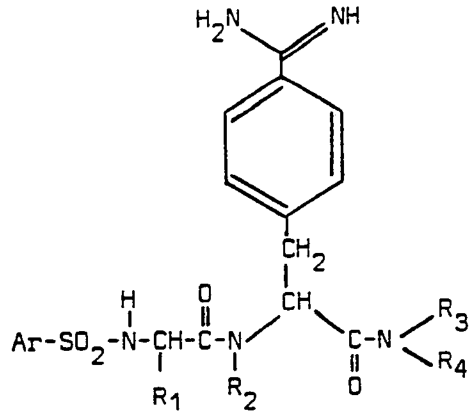

(I)

dans laquelle:

$R_1$ représente l'hydrogène, un groupe alcoyle ou hydroxyalcoyle en $C_1$—$C_6$, benzyle, un groupe phényle ou un groupe hydroxy-4 phényle;

$R_2$ représente un groupe alcoyle, alcényle ou alcynyle ayant jusqu'à 6 atomes de carbone, un groupe benzyle, un groupe alcoxy (en $C_1$—$C_6$) carbonylalcoyle en $C_1$—$C_6$, carboxyalcoyle en $C_1$—$C_6$, hydroxyalcoyle en $C_1$—$C_6$;

$R_3$ et $R_4$, identiques ou différents, représentent chacun un radical alcoyle, hydroxyalcoyle alcényle ou alcynyle ayant jusqu'à 6 atomes de carbone, ou forment ensemble, avec l'azote auquel ils sont rattachés, un hétérocycle sature choisi parmi morpholino, thiomorpholino, pyrrolidino non substitué ou substitué par un groupe alcoxy ($C_1$—$C_6$) carbonyle ou carboxy, pipérazino, (alcoyle en $C_1$—$C_6$)-4 pipérazino, (hydroxyalcoyle en $C_1$—$C_6$)-4 pipérazino, ou pipéridino non substitué ou substitué par un groupe alcoyle en $C_1$—$C_6$, benzyle, hydroxy, hydroxyalcoyle en $C_1$—$C_6$, amino, aminoalcoyle en $C_1$—$C_6$, hydroxyamino, alcoxy ($C_1$—$C_6$) carbonyle ou carboxy;

Ar représente un groupe phényle, alpha-naphtyle, béta-naphtyle, méthoxycarbonyl-phényle, ou bien un groupe hétéroaryle choisi parmi les radicaux pyridyle, quinoléinyle, isoquinoléinyle ou methylquinoléinyle; et leurs sels d'addition avec les acides minéraux ou organiques pharmaceutiquement acceptables ainsi que les stéréoisomères ou leur mélange; caractérisé en ce que l'on fait réagir sur la cyano-4 phénylalaninamide Nα-alcoylée de formule (II),

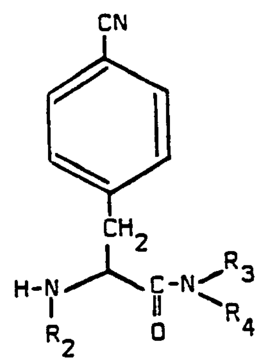

(II)

dans laquelle $R_2$, $R_3$ et $R_4$ ont les mêmes significations que dans la formule (I), une acide de formule

$$Ar—SO_2—NH—\underset{\displaystyle R_1}{\underset{|}{CH}}—COOH \qquad (III)$$

sous sa forme activée

$$Ar—SO_2—NH—\underset{\displaystyle R_1}{\underset{|}{CH}}—CO—R \qquad (IV)$$

dans laquelle Ar et $R_1$ ont les mêmes significations que dans la formule (I) et R représente un bon groupement nucléofuge, tel que chlore alcoxycarbonyloxy ou hétéroaryle, pour obtenir le composé de formule (V)

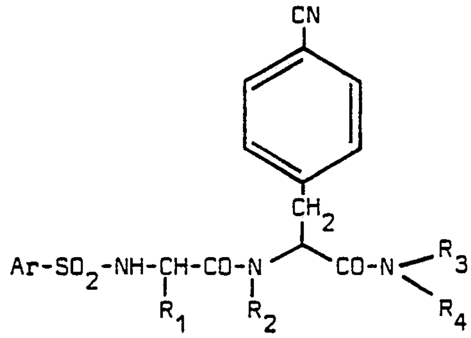

(V)

dans laquelle Ar, $R_1$, $R_2$, $R_3$ et $R_4$ ont les mêmes significations que dans la formule (I) qu'on traite avec un excès d'une solution saturée de gaz chlorhydrique dans un alcool de formule X—OH dans laquelle X représente un radical alcoyle inférieur, pour obtenir l'imidoester de formule (VI) sous forme de chlorhydrate

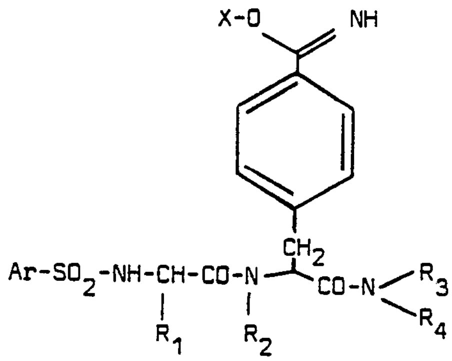

(VI)

dans laquelle Ar, $R_1$, $R_2$, $R_3$, $R_4$ et X ont les mêmes significations précitées qui est alors traité par un excès d'une solution de gaz ammoniac dans un alcool inférieur à la température d'ébullition du mélange réactionnel pour obtenir le composé de formule (I) recherché.

2. Procédé selon la revendication 1, caractérisé en ce que l'activation de l'acide de formule (III) est effectuée par action d'un chloroformate d'alcoyle.

3. Procédé selon la revendication 1, caractérisé en ce que l'activation de l'acide de formule (III) est effectuée par action de réactifs de couplage n'induisant pas la racémisation, tels que l'hydroxy-1 benzotriazole en présence de N,N dicyclohexylcarbodiimide, l'hexafluorophosphate de benzotriazolyl-1 oxytris (diméthylamino) phosphonium ou le chlorure de N,N-bis (oxo-2 oxazolidinyl-3) phosphorodiamidique.

4. Procédé selon l'une des revendications 1 à 3, caractérisé en ce que la préparation de l'imidoester de formule (VI) s'effectue en milieu alcoolique, en présence d'un acide fort.

5. Procédé selon l'une des revendications 1 à 4, caractérisé en ce que la formation de l'amidine de formule (I) s'effectue en milieu alcoolique, par action du gaz ammoniac.

6. Procédé de préparation d'un médicament, caractérisé en ce que l'on utilise, à titre de principe actif, un dérivé de formule (I) obtenu suivant l'une des revendications 1 à 5 ou l'un de ses sels pharmaceutiquement acceptables.

7. Procédé de préparation de composés de formule (II):

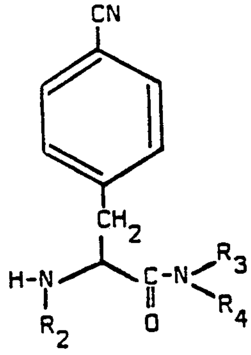

(II)

dans laquelle $R_2$, $R_3$ et $R_4$ ont la même signification que dans la revendication 1, ainsi que les isomères ou leurs mélanges et leurs sels d'acides minéraux ou organiques, caractérisé en ce que l'on fait réagir l'acide de formule (IX) activée, par l'action de réactifs de couplage n'induisant pas la racémisation tels que l'hydroxy-1 benzotriazole en présence de N,N-dicyclohéxylcarbodiimide, l'héxafluorophosphate de benzotriazolyl-1 oxytris (diméthylamino) phosphonium ou le chlorure de N,N-bis (oxo-2 oxazolidinyl-3) phosphorodiamidique, em composé de formule (X)

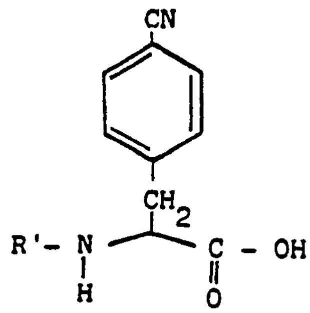 (IX) 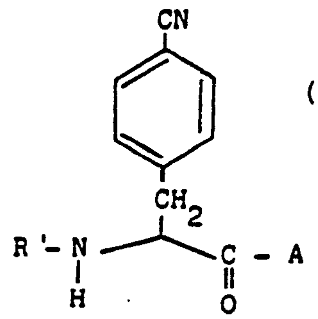 (X)

dans lesquelles R' représente un groupement N-protecteur et A représente le reste du réactif de couplage, avec l'amine de formule

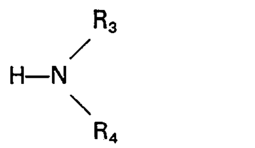

(XI) dans laquelle $R_3$ et $R_4$ ont les mêmes significations que dans la formule II, pour former le composé de formule:

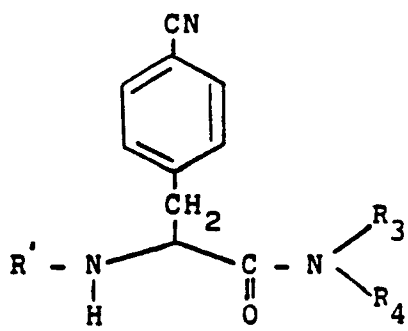

(XII)

dans laquelle R', $R_2$ et $R_4$ ont les mêmes significations que dans les formules II et IX, que l'on fait réagir avec un composé $R_2$—X, dans lequel X est un halogéne et $R_2$ est tel que dans la formule II, pour obtenir le composé de formule

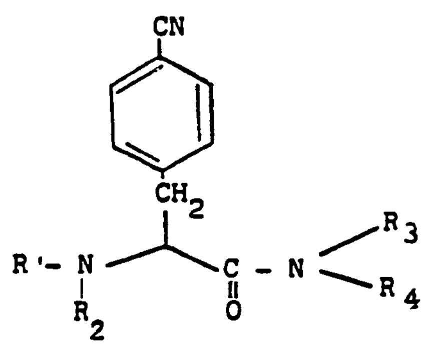

(XIII)

qui, par clivage du groupement protecteur R', conduit aux composés de formule (II).

**Patentansprüche für die Vertragsstaaten: BE CH DE FR GB IT LI LU NL SE**

1. Verbindungen der Formel

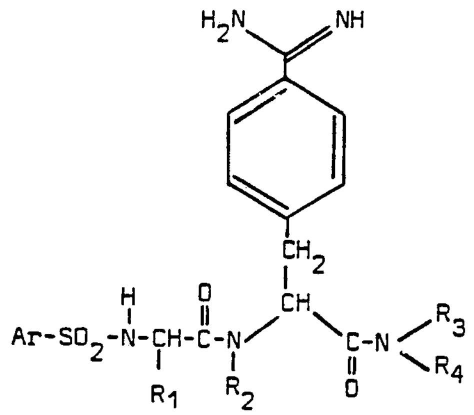

(I)

in der bedeuten:

$R_1$ Wasserstoff, $C_1$—$C_6$-Alkyl oder $C_1$—$C_6$-Hydroxyalkyl, Benzyl, Phenyl oder 4-Hydroxyphenyl;

$R_2$ Alkyl, Alkenyl oder Alkinyl mit jeweils bis zu 6 Kohlenstoffatomen, Benzyl, Alcoxy-($C_1$—$C_6$)-carbonyl-$C_1$—$C_6$-alkyl, Carboxy-($C_1$—$C_6$)-alkyl oder $C_1$—$C_6$-Hydroxyalkyl;

$R_3$ und $R_4$ zugleich oder unabhängig Alkyl Hydroxyalkyl, Alkenyl oder Alkinyl mit jeweils bis zu 6 Kohlenstoffatomen oder zusammen mit dem Stickstoffatom, an dem sie gebunden sind, einen gesättigten Heterocyclus, der ausgewählt ist unter Moropholino, Thiomorpholino, Pyrrolidino, das ggfs. mit einer Alkoxy-($C_1$—$C_6$)-carbonylgruppe oder Carboxylgruppe substituiert ist, Piperazino, 4-($C_1$—$C_6$-Alkyl)-substituiertes Piperazino, 4-($C_1$—$C_6$-Hydroxyalkyl)-substituiertes Piperazino oder Piperidino, das ggfs. mit $C_1$—$C_6$-Alkyl, Benzyl, Hydroxy, $C_1$—$C_6$-Hydroxyalkyl, Amino, $C_1$—$C_6$-Aminoalkyl, Alkoxy-($C_1$—$C_6$)-carbonyl oder Carboxyl substituiert ist, und

Ar Phenyl, α-Naphthyl, β-Naphthyl, Methoxycarbonylphenyl oder eine Heteroarylgruppe, die ausgewählt ist unter Pyridyl, Chinolinyl, Isochinolinyl oder Methylchinolinyl, sowie ihre Additionssalze mit pharmazeutisch akzeptablen anorganischen oder organischen Säuren sowie ihre Stereoisomeren oder deren Gemische.

2. Verbindungen der Formel I nach Anspruch 1, in der $R_1$ Wasserstoff oder Alkyl bedeutet.

3. Verbindungen der Formel I nach Anspruch 1 oder 2, in der $R_2$ Alkyl bedeutet.

4. Verbindungen der Formel I nach Anspruch 1, 2 oder 3, in der die Gruppe

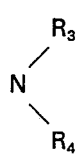

Piperidino bedeutet, das ggfs. mit Methyl oder Benzyl substituiert ist.

5. Verbindungen der Formel I nach Anspruch 1, 2, 3 order 4, in der Ar Naphthyl bedeutet.

6. 1-[Nα-Methyl-Nα-(N-β-naphthylsulfonylglycyl)-p-amidinophenylalanyl]-piperidin und seine pharmazeutisch akzeptablen Salze.

7. 1-Methyl-4-[Nα-methyl-Nα-(N-β-naphthylsulfonylglycyl)-p-amidinophenylalanyl]-piperidin und seine pharmazeutisch akzeptablen Salze.

8. 1-[Nα-Ethyl-Nα-(N-β-naphthylsulfonylglycyl)-p-amidinophenylalanyl]-piperidin und seine pharmazeutisch akzeptablen Salze.

9. 1-[Nα-Ethyl-Nα-(N-β-naphthylsulfonylglycyl)-p-amidinophenylalanyl]-4-methylpiperidin und seine pharmazeutisch akzeptablen Salze.

10. 1-[Nα-Methyl-Nα-(N-chinolinyl-8-sulfonylglycyl)-p-amidinophenylalanyl]-piperidin und seine pharmazeutisch akzeptablen Salze.

11. 1-[Nα-Methyl-Nα-(N-β-naphthylsulfonyl-(S)-seryl)-p-amidinophenylalanyl]-piperidin und seine pharmazeutisch akzeptablen Salze.

12. 1-[Nα-Methyl-Nα-(N-β-naphthylsulfonyl-(S)-threonyl)--p-amidinophenylalanyl]-piperidin und seine pharmazeutisch akzeptablen Salze.

13. Verfahren zur Herstellung der Verbindungen nach den Ansprüchen 1 bis 12, gekennzeichnet durch Umsetzung des Nα-alkylierten 4-Cyanophenylalaninamids der Formel II

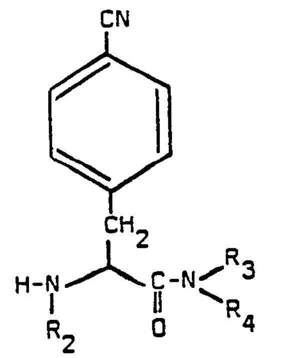

(II)

mit $R_2$, $R_3$ und $R_4$ wie oben in Formel I mit einer Säure der Formel

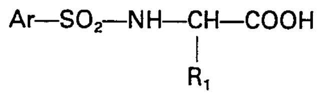

(III)

in ihrer aktivierten Form

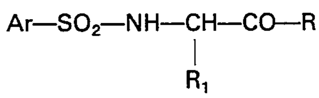

(IV)

in der Ar und $R_1$ die obige Bedeutung wie in Formel I besitzen und

R eine geeignete nucleofuge Gruppe wie Chlor, Alkoxycarbonyloxy oder Heteroaryl darstellt, zur Verbindung der Formel V

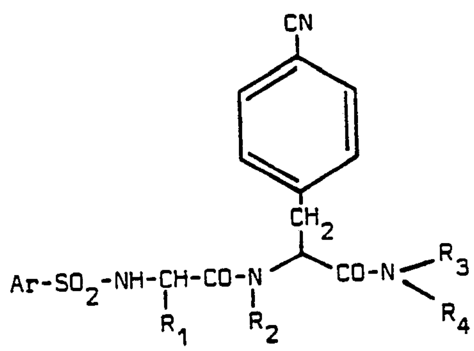

(V)

mit Ar, $R_1$, $R_2$, $R_3$ und $R_4$ wie oben in Formel I, Behandlung der erhaltenen Verbindung mit einem Überschuß einer mit gasförmigem Chlorwasserstoff gesättigten Lösung in einem Alkohol der Formel X—OH, in der X niederes Alkyl darstellt, unter Erhalt des Imidoesters der Formel VI in Form des Hydrochlorids

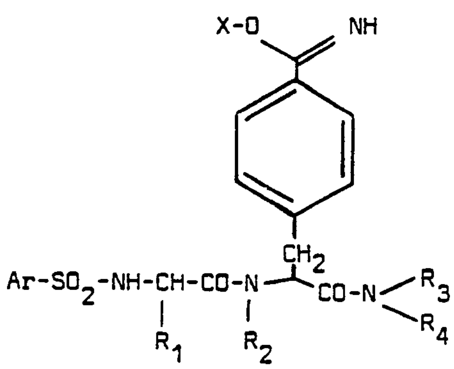

(VI)

mit Ar, $R_1$, $R_2$, $R_3$, $R_4$ und X wie oben und Behandlung mit einem Überschuß einer Lösung von gasförmigem Ammoniak in einem niederen Alkohol bei der Siedetemperatur des Reaktionsgemischs unter Erhalt der angestrebten Verbindung der Formel I.

14. Verfahren nach Anspruch 13, dadurch gekennzeichnet, daß die Aktivierung der Säure der Formel III durch Einwirkung eines Alkylchloroformiats vorgenommen wird.

15. Verfahren nach Anspruch 13, dadurch gekennzeichnet, daß die Aktivierung der Säure der Formel II durch Einwirkung von Kopplungsreagentien, die nicht zu einer Racemisierung führen, wie 1-Hydroxybenzotriazol in Gegenwart, von N,N-Dicyclohexylcarbodiimid, 1-Benzotriazolyl-oxy-tris(dimethylamino)-phosphoniumhexafluorphosphat oder N,N-Bis(2-oxo-3-oxazolidinyl)-phosphordiamid-chlorid, vorgenommen wird.

16. Verfahren nach einem der Ansprüche 13 bis 15, dadurch gekennzeichnet, daß die Herstellung des Imidoesters der Formel VI in einem alkoholischen Medium in Gegenwart einer starken Säure vorgenommen wird.

17. Verfahren nach einem der Ansprüche 13 bis 16, dadurch gekennzeichnet, daß die Erzeugung des Amidins der Formel I in einem alkoholischen Medium durch Einwirkung von gasförmigem Ammoniak vorgenommen wird.

18. Pharmazeutische Mittel, dadurch gekennzeichnet, daß sie ein Derivat der Formel I nach einem der Ansprüche 1 bis 12 oder eines seiner pharmazeutisch akzeptablen Salze also Wirkstoff enthalten.

19. Pharmazeutische Mittel nach Anspruch 18, dadurch gekennzeichnet, daß sie in Dosiereinheiten vorliegen, die jeweils 0,005 bis 0,500 g Wirkstoff enthalten.

20. Verbindungen der Formel II

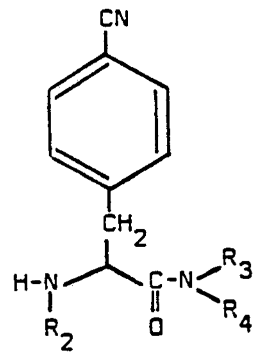

(II)

mit $R_2$, $R_3$ und $R_4$ wie in Anspruch 1 sowie ihre Isomeren oder deren Gemische und ihre Salze mit anorganischen oder organischen Säuren.

21. Verfahren zur Herstellung der Verbindungen der Formel II nach Anspruch 20, gekennzeichnet durch Umsetzung der Säure der Formel IX, die durch Einwirkung von Kopplungsreagentien, die nicht zu einer Racemisierung führen, wie 1-Hydroxybenzotriazol in Gegenwart von N,N-Dicyclohexylcarbodiimid, 1-Benzotriazolyl-oxy-tris(dimethylamino)-phosphonium-hexafluorphosphat oder N,N-Bis(2-oxo-3-oxazolidinyl)-phosphordiamid-chlorid, zu einer Verbindung der Formel X aktiviert wurde,

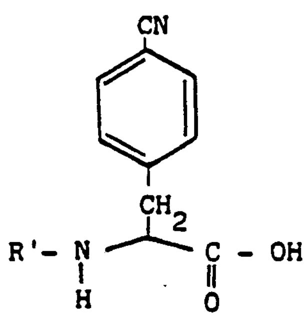

(IX)

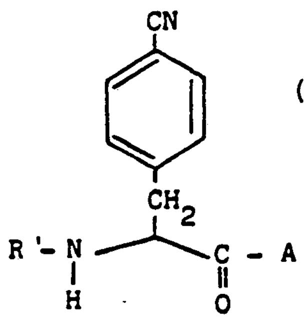

(X)

worin R' eine N-Schutzgruppe und A den Rest des Kopplungsreagens bedeuten, mit einem Amin der Formel

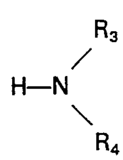

(XI)

mit $R_3$ und $R_4$ wie oben in Formel II zur Verbindung der Formel

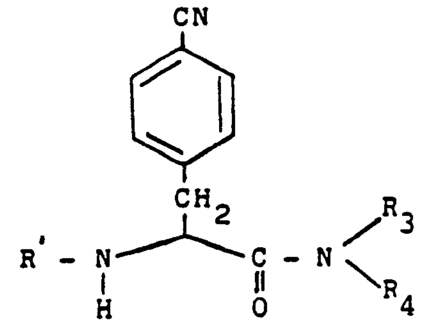

(XII)

mit R', $R_2$ und $R_4$ wie in den Formeln II und IX und Umsetzung dieser Verbindung mit einer Verbindung der Formel $R_2$—X, in der X ein Halogen und $R_2$ dasselbe wie in Formel II bedeuten, zur Verbindung der Formel

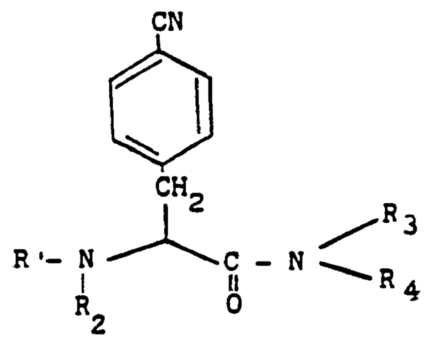

(XIII)

die nach Abspaltung der Schutzgruppe R' die entsprechende Verbindung der Formel II ergibt.

**Patentansprüche für die Vertragsstaaten: AT GR ES**

1. Verfahren zur Herstellung von Verbindungen der Formel

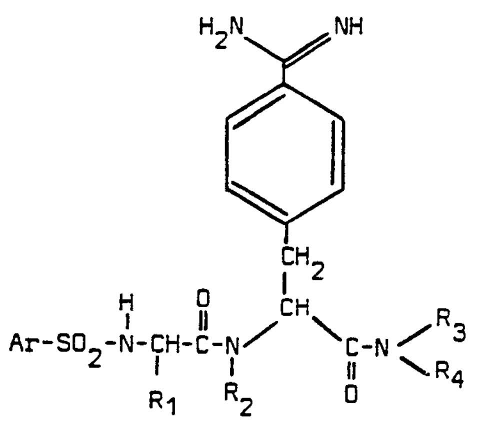

(I)

in der bedeuten:

$R_1$ Wasserstoff, $C_1$—$C_6$-Alkyl oder $C_1$—$C_6$-Hydroxyalkyl, Benzyl, Phenyl oder 4-Hydroxyphenyl;

$R_2$ Alkyl, Alkenyl oder Alkinyl mit jeweils bis zu 6 Kohlenstoffatomen, Benzyl, Alcoxy-($C_1$—$C_6$)-carbonyl-$C_1$—$C_6$-alkyl, Carboxy-($C_1$—$C_6$)-alkyl oder $C_1$—$C_6$-Hydroxyalkyl;

$R_3$ und $R_4$ zugleich oder unabhängig Alkyl Hydroxyalkyl, Alkenyl oder Alkinyl mit jeweils bis zu 6 Kohlenstoffatomen oder zusammen mit dem Stickstoffatom, an dem sie gebunden sind, einen gesättigten Heterocyclus, der ausgewählt ist unter Moropholino, Thiomorpholino, Pyrrolidino, das ggfs. mit einer Alkoxy-($C_1$—$C_6$)-carbonylgruppe oder Carboxylgruppe substituiert ist, Piperazino, 4-($C_1$—$C_6$-Alkyl)-substituiertes Piperazino, 4-($C_1$—$C_6$-Hydroxyalkyl)-substituiertes Piperazino oder Piperidino, das ggfs. mit $C_1$—$C_6$-Alkyl, Benzyl, Hydroxy, $C_1$—$C_6$-Hydroxyalkyl, Amino, $C_1$—$C_6$-Aminoalkyl, Alkoxy-($C_1$—$C_6$)-carbonyl oder Carboxyl substituiert ist, und

Ar Phenyl, α-Naphthyl, β-Naphthyl, Methoxycarbonylphenyl oder eine Heteroarylgruppe, die ausgewählt ist unter Pyridyl, Chinolinyl, Isochinolinyl oder Methylchinolinyl, sowie ihre Additionssalze mit pharmazeutisch akzeptablen anorganischen oder organischen Säuren sowie ihre Stereoisomeren oder deren Gemische, gekennzeichnet durch Umsetzung des Nα-alkylierten 4-Cyanophenylalaninamids der Formel II

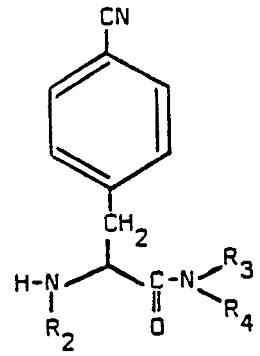

(II)

mit $R_2$, $R_3$ und $R_4$ wie oben in Formel I mit einer Säure der Formel

$$Ar{-}SO_2{-}NH{-}\underset{\displaystyle R_1}{\underset{|}{CH}}{-}COOH \qquad (III)$$

in ihrer aktivierten Form

$$Ar{-}SO_2{-}NH{-}\underset{\displaystyle R_1}{\underset{|}{CH}}{-}CO{-}R \qquad (VI)$$

in der Ar und $R_1$ die obige Bedeutung wie in Formel I besitzen und

R eine geeignete nucleofuge Gruppe wie Chlor, Alkoxycarbonyloxy oder Heteroaryl darstellt, zur Verbindung der Formel V

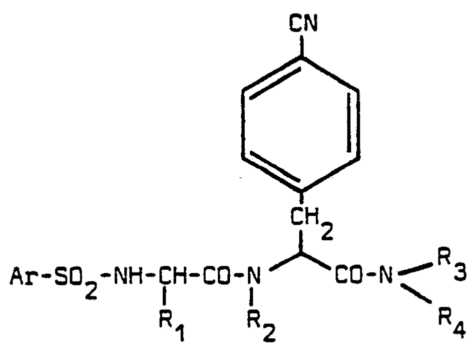

(V)

mit Ar, $R_1$, $R_2$, $R_3$ und $R_4$ wie oben in Formel I, Behandlung der erhaltenen Verbindung mit einem Überschuß einer mit gasförmigem Chlorwasserstoff gesättigten Lösung in einem Alkohol der Formel X—OH, in der X niederes Alkyl darstellt, unter Erhalt des Imidoesters der Formel VI in Form des Hydrochlorids

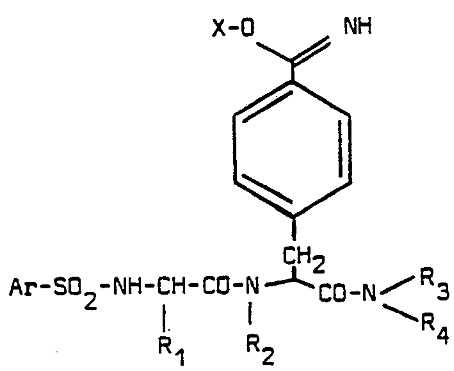

(VI)

mit Ar, $R_1$, $R_2$, $R_3$, $R_4$ und X wie oben und Behandlung mit einem Überschuß einer Lösung von gasförmigem Ammoniak in einem niederen Alkohol bei der Siedetemperatur des Reaktionsgemischs unter Erhalt der angestrebten Verbindung der Formel I.

2. Verfahren nach Anspruch 1, dadurch gekennzeichnet, daß die Aktivierung der Säure der Formel III durch Einwirkung eines Alkylchlorformiats vorgenommen wird.

3. Verfahren nach Anspruch 1, dadurch gekennzeichnet, daß die Aktivierung der Säure der Formel III durch Einwirkung von Kopplungsreagentien, die nicht zu einer Racemisierung führen, wie 1-Hydroxybenzotriazol in Gegenwart von N,N-Dicyclohexylcarbodiimid, 1-Benzo-triazolyl-oxy-tris(dimethylamino)-phosphoniumhexafluorphosphat oder N,N-Bis(2-oxo-3-oxazolidinyl)-phosphordiamid-chlorid, vorgenommen wird.

4. Verfahren nach einem der Ansprüche 1 bis 3, dadurch gekennzeichnet, daß die Herstellung des Imidoesters der Formel VI in einem alkoholischen Medium in Gegenwart einer starken Säure vorgenommen wird.

5. Verfahren nach einem der Ansprüche 1 bis 4, dadurch gekennzeichnet, daß die Erzeugung des Amidins der Formel I in einem alkoholischen Medium durch Einwirkung von gasförmigem Ammoniak vorgenommen wird.

6. Verfahren zur Herstellung pharmazeutischer Mittel, gekennzeichnet durch Verwendung eines nach einem der Ansprüche 1 bis 5 erhaltenen Derivats der Formel I oder eines seiner pharmazeutisch akzeptablen Salze als Wirkstoff.

7. Verfahren zur Herstellung von Verbindungen der Formel II

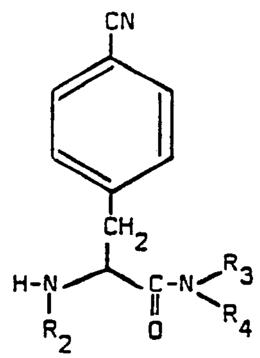

(II)

mit $R_2$, $R_3$ und $R_4$ wie in Anspruch 1 sowie ihrer Isomeren oder deren Gemische und ihrer Salze mit anorganischen oder organischen Säuren, gekennzeichnet durch Umsetzung der Säure der Formel XI, die durch Einwirkung von Kopplungsreagentien, die nicht zu einer Racemisierung führen, wie 1-Hydroxybenzotriazol in Gegenwart von N,N-Dicyclohexylcarbodiimid, 1-Benzotriazolyl-oxy-tris(dimethylamino)-phosphonium-hexafluorphosphat oder N,N-Bis(2-oxo-3-oxazolidinyl)-phosphordiamid-chlorid zu einer Verbindung der Formel X aktiviert wurde,

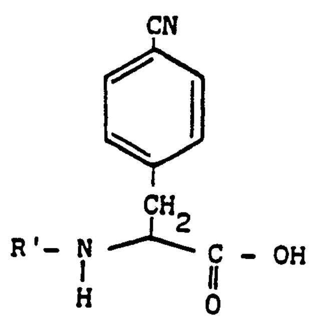

(IX)

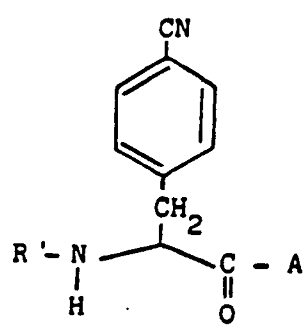

(X)

worin R' eine N-Schutzgruppe und A den Rest des Kopplungsreagens bedeuten, mit einem Amin der Formel

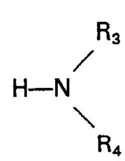

(XI)

mit $R_3$ und $R_4$ wie oben in Formel II zur Verbindung der Formel

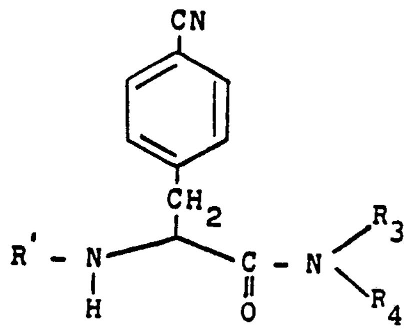

(XII)

mit R', $R_2$ und $R_4$ wie in den Formeln II und IX und Umsetzung dieser Verbindung mit einer Verbindung der Formel $R_2$—X, in der X ein Halogen und $R_2$ dasselbe wie in Formel II bedeuten, zur Verbindung der Formel

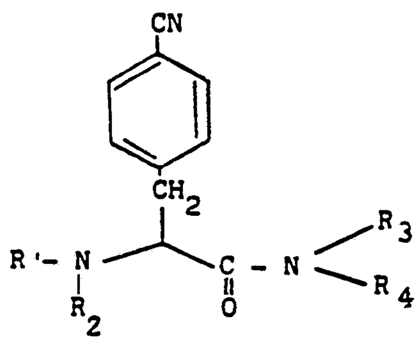

(XIII)

die nach Abspaltung der Schutzgruppe R' die entsprechende Verbindung der Formel II ergibt.

**Claims for the Contracting States: BE CH DE FR GB IT LI LU NL SE**

1. Compounds of formula

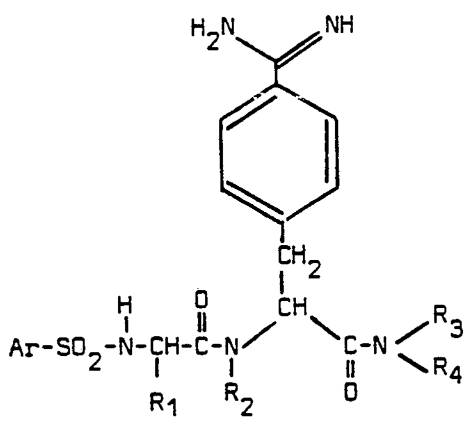

(I)

wherein

$R_1$ represents hydrogen, a $C_{1-6}$ alkyl or hydroxyalkyl group, benzyl, a phenyl group or a 4-hydroxyphenyl group;

$R_2$ represents an alkyl, alkenyl or alkynyl group each having up to 6 carbon atoms, a benzyl group, a $C_{1-6}$ alkoxy, $C_{1-6}$ carbonylalkyl, $C_{1-6}$ carboxyalkyl or $C_{1-6}$ hydroxyalkyl group;

$R_3$ und $R_4$, which may be identical or different, each represent an alkyl, hydroxyalkyl, alkenyl or alkynyl group each having up to 6 carbon atoms, or form together with the nitrogen to which they are attached a saturated heterocyclic group selected from among morpholino, thiomorpholino, pyrrolidino, either unsubstituted or substituted by a $C_{1-6}$ alkoxy, carbonyl or carboxy group, piperazino, 4-($C_{1-6}$ alkyl)-piperazino, 4-($C_{1-6}$ hydroxyalkyl)-piperazino, or piperidino with either unsubstituted or substituted by a

$C_{1-6}$ alkyl group, benzyl, hydroxy, $C_{1-6}$ hydroxyalkyl, amino, $C_{1-6}$ aminoalkyl, hydroxyamino, $C_{1-6}$ alkoxy, carbonyl or carboxy;

Ar represents a phenyl, alpha-naphthyl, beta-naphthyl, methoxycarbonylphenyl group or a heteroaryl group selected from among the pyridyl, quinolinyl, isoquinolinyl or methyl-quinolinyl groups; and the addition salts thereof with pharmaceutically acceptable organic or inorganic acids and the stereoisomers or mixtures thereof.

2. Compounds according to claim 1 of formula (I), wherein $R_1$ represents hydrogen or an alkyl radical.

3. Compounds according to claim 1 or 2 of formula (I) wherein $R_2$ represents an alkyl radical.

4. Compounds according to claim 1, 2 or 3, of formula (I), wherein the group

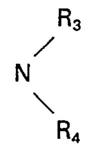

represents a piperidino radical either unsubstituted or substituted by a methyl or benzyl group.

5. Compounds according to claim 1, 2, 3 or 4, of formula (I), wherein Ar represents a naphthyl radical.

6. 1-[Nα-methyl-Nα-(N-betanaphthylsulonylglycyl)-p-amidinophenylalanyl]-piperidine and the pharmaceutically acceptable salts thereof.

7. 4-Methyl-1-[Nα-methyl-Nα(N-betanaphthylsulphonylglycyl)-p-amidinophenylalanyl]-piperidine and the pharmaceutically acceptable salts thereof.

8. 1-[Nα-ethyl-Nα-(N-betanaphthylsulphonylglycyl)-p-amidinophenylalanyl]-piperidine and the pharmaceutically acceptable salts thereof.

9. 1-[Nα-ethyl-Nα-(N-betanaphthylsulphonylglycyl)-p-amidinophenylalanyl]-4-methyl-piperidine and the pharmaceutically acceptable salts thereof.

10. 1-[Nα-methyl-Nα-(N-quinolinyl-8-sulphonylglycyl)-p-amidinophenylalanyl]-piperidine and the pharmaceutically acceptable salts thereof.

11. 1-[Nα-methyl-Nα-(betanaphthylsulphonyl-(S)-seryl)-p-amidinophenylalanyl]-piperidine and the pharmaceutically acceptable salts thereof.

12. 1-[Nα-methyl-Nα-(N-betanaphthylsulphonyl-(S)-threonyl)-p-amidinophenylalanyl]-piperidine and the pharmaceutically acceptable salts thereof.

13. Process for preparing compounds according to claims 1 to 12, characterized in that the Nα-alkylated 4-cyanophenylalaninamide of formula (II)

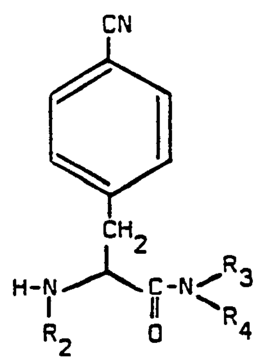

(II)

wherein $R_2$, $R_3$ and $R_4$ have the same meanings as in formula (I), is reacted with an acid of formula

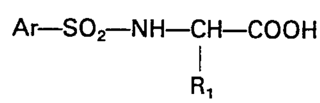

(III)

in its activated form

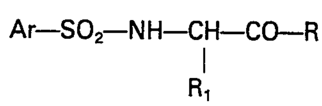

(IV)

wherein Ar and $R_1$ have the same meanings as in formula (I) and R represents a good nucleophobic group such as alkoxycarbonyloxy or heteroaryl chlorine, to obtain the compound of formula (V)

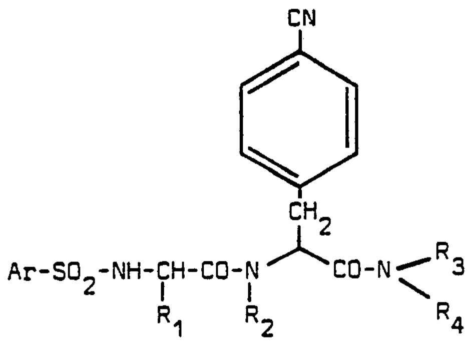

(V)

wherein Ar, $R_1$, $R_2$, $R_3$ and $R_4$ have the same meanings as in formula (I), which is treated with an excess of a saturated solution of hydrochloric gas in an alcohol of formula X—OH wherein X represents a lower alkyl radical, in order to obtain the imidoester of formula (VI) in the form of the hydrochloride

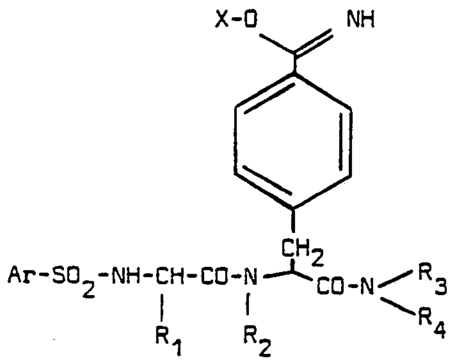

(VI)

wherein Ar, $R_1$, $R_2$, $R_3$, $R_4$ and X have the meanings given hereinbefore, which is then treated with an excess of a solution of ammonia gas in a lower alcohol at the boiling temperature of the reaction mixture in order to obtain the desired compound of formula (I).

14. Process according to claim 13, characterised in that the acid of formula (III) is activated by the action of an alkyl chloroformate.

15. Process according to claim 13, characterised in that the acid of formula (III) is activated by the action of coupling reagents which do not cause racemisation, such as 1-hydroxybenzotriazole in the presence of N,N-dicyclohexylcarbodiimide, 1-benzotriazolyl-oxytris-(dimethylamino)-phosphonium hexafluorophosphate or N,N-bis-(2-oxo-3-oxazolidinyl)-phosphorodiamide chloride.

16. Process according to one of claims 13 to 15, characterised in that the imidoester of formula (VI) is prepared in an alcoholic medium, in the presence of a strong acid.

17. Process according to one of claims 13 to 16, characterised in that the amidine of formula (I) is prepared in an alcoholic medium, by the action of ammonia gas.

18. Drug, characterised in that it contains as active principle a derivative of formula (I) according to one of claims 1 to 12 or a pharmaceutically acceptable salt thereof.

19. Drug according to claim 19, characterised in that it is present in the form of single doses each containing from 0.005 g to 0.500 g of active principle.

20. Compounds of formula (II)

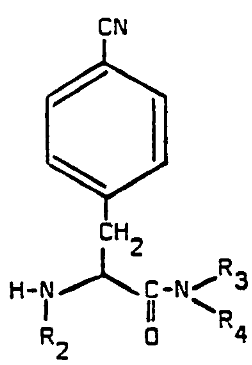

(II)

wherein $R_2$, $R_3$ and $R_4$ have the same meanings as in claim 1, and the isomers or mixtures thereof and the inorganic or organic acid salts thereof.

21. Process for preparing compounds of formula (II) according to claim 20, characterised in that the acid of formula (IX), activated by the action of coupling reagents which do not cause racemisation, such as 1-hydroxybenzotriazole in the presence of N,N-dicyclohexylcarbodiimide, 1-benzatriazolyloxytris-(dimethylamino)-phosphonium hexafluorophosphate or N,N-bis-(2-oxo-3-oxazolidinyl)-phosphorodiamide chloride, into a compound of formula (X)

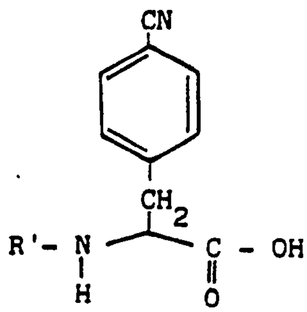
(IX)

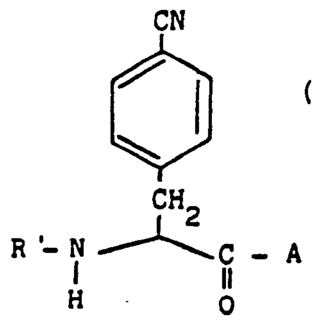
(X)

wherein R′ represents an N-protecting group and A represents the remainder of the coupling reagent, is reacted with the amine of formula

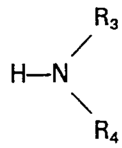
(XI)

wherein $R_3$ and $R_4$ have the same meanings as in formula II, to form the compound of formula:

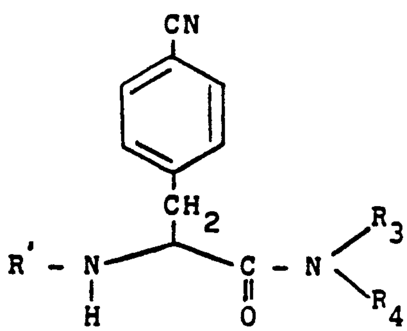
(XII)

wherein R′, $R_2$ and $R_4$ have the same meanings as in formulae II and IX, which is then reacted with a compound $R_2$—X, wherein X is a halogen and $R_2$ is as defined in formula II, to obtain the compound of formula

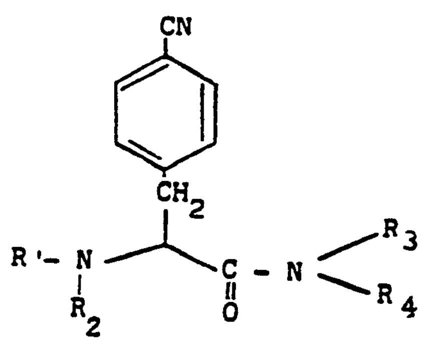
(XIII)

which, by cleaving of the protecting group R′, yields the compounds of formula (II).

**Claims for the Contracting States: AT GR ES**

1. Process for preparing compounds of formula

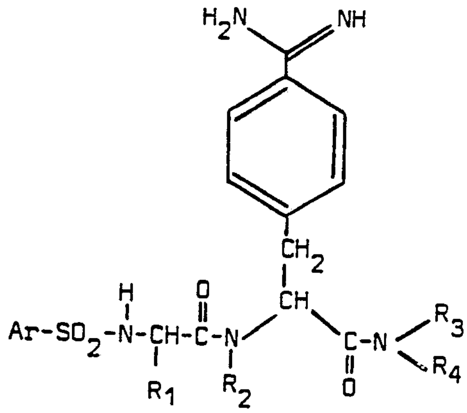

(I)

wherein

$R_1$ represents hydrogen, a $C_{1-6}$ alkyl or hydroxyalkyl group, benzyl, a phenyl group or a 4-hydroxyphenyl group;

$R_2$ represents an alkyl, alkenyl or alkynyl group each having up to 6 carbon atoms, a benzyl group, a $C_{1-6}$ alkoxy, $C_{1-6}$ carbonylalkyl, $C_{1-6}$ carboxyalkyl or $C_{1-6}$ hydroxyalkyl group;

$R_3$ und $R_4$, which may be identical or different, each represent an alkyl, hydroxyalkyl, alkenyl or alkynyl group each having up to 6 carbon atoms, or form together with the nitrogen to which they are attached a saturated heterocyclic group selected from among morpholino, thiomorpholino, pyrrolidino, either unsubstituted or substituted by a $C_{1-6}$ alkoxy, carbonyl or carboxy group, piperazino, 4-($C_{1-6}$ alkyl)-piperazino, 4-($C_{1-6}$ hydroxyalkyl)-piperazino, or piperidino with either unsubstituted or substituted by a $C_{1-6}$ alkyl group, benzyl, hydroxy, $C_{1-6}$ hydroxyalkyl, amino, $C_{1-6}$ aminoalkyl, hydroxyamin, $C_{1-6}$ alkoxy, carbonyl or carboxy;

Ar represents a phenyl, alpha-naphthyl, beta-naphthyl, methoxycarbonylphenyl group or a heteroaryl group selected from among the pyridyl, quinolinyl, isoquinolinyl or methyl-quinolinyl groups; and the addition salts thereof with pharmaceutically acceptable organic or inorganic acids and the stereoisomers or mixtures thereof, characterized in that the Nα-alkylated 4-cyano-phenylalaninamide of formula (II)

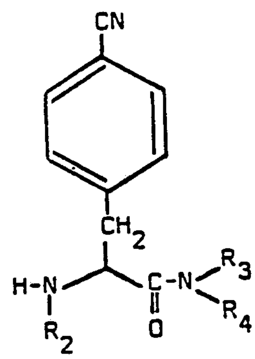

(II)

wherein $R_2$, $R_3$ and $R_4$ have the same meanings as in formula (I), is reacted with an acid of formula

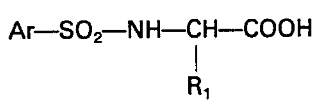

(III)

in its activated form

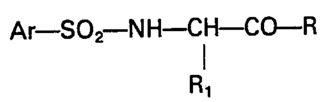

(IV)

wherein Ar and $R_1$ have the same meanings as in formula (I) and R represents a good nucleophobic group such as alkoxycarbonyloxy or heteroaryl chlorine, to obtain the compound of formula (V)

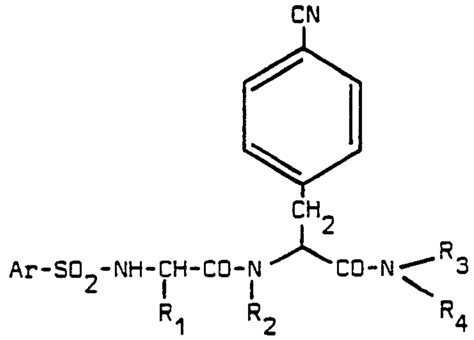

(V)

wherein Ar, $R_1$, $R_2$, $R_3$ and $R_4$ have the same meanings as in formula (I), which is treated with an excess of a saturated solution of hydrochloric gas in an alcohol of formula X—OH wherein X represents a lower alkyl radical, in order to obtain the imidoester of formula (VI) in the form of the hydrochloride

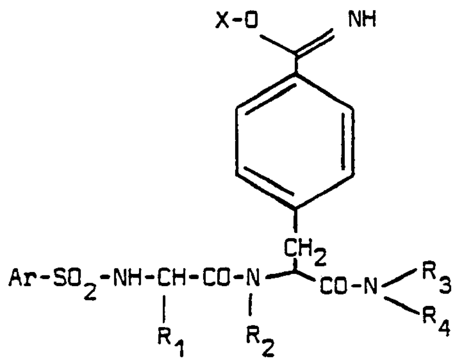

(VI)

wherein Ar, $R_1$, $R_2$, $R_3$, $R_4$ and X have the meanings given hereinbefore, which is then treated with an excess of a solution of ammonia gas in a lower alcohol at the boiling temperature of the reaction mixture in order to obtain the desired compound of formula (I).

2. Process according to claim 1, characterised in that the acid of formula (III) is activated by the action of an alkyl chloroformate.

3. Process according to claim 1, characterised in that the acid of formula (III) is activated by the action of coupling reagents which do not cause racemisation, such as 1-hydroxybenzotriazole in the presence of N,N-dicyclohexylcarbodiimide, 1-benzo-triazolyl-oxytris-(dimethylamino)-phosphonium hexafluorophosphate or N,N-bis-(2-oxo-3-oxazolidinyl)-phosphorodiamide chloride.

4. Process according to one of claims 1 to 3, characterised in that the imidoester of formula (VI) is prepared in an alcoholic medium, in the presence of a strong acid.

5. Process according to one of claims 1 to 4, characterised in that the amidine of formula (I) is prepared in an alcoholic medium, by the action of ammonia gas.

6. Process for preparing a drug, characterised in that a derivative of formula (I) obtained according to one of claims 1 to 5 or a pharmaceutically acceptable salt thereof is used as active principle.

7. Process for preparing compounds of formula (II):

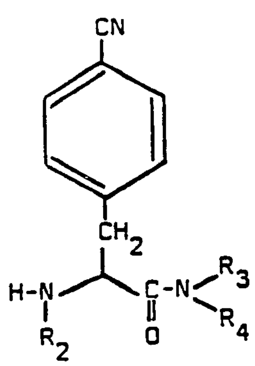

(II)

wherein $R_2$, $R_3$ and $R_4$ have the same meaning as in claim 1, and the isomers or mixtures thereof and the inorganic or organic acid salts thereof, characterised in that the acid of formula (IX) activated, by the action

of coupling reagents which do not cause racemisation, such as 1-hydroxybenzotriazole in the presence of N,N-dicyclohexylcarbodiimide, 1-benzotriazolyl-oxytris-(dimethylamino)-phosphonium hexafluorophosphate or N,N-bis-(2-oxo-3-oxazolidinyl)-phosphorodiamide chloride, into a compound of formula (X)

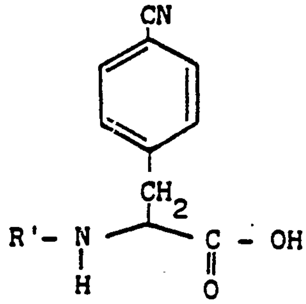

(IX)

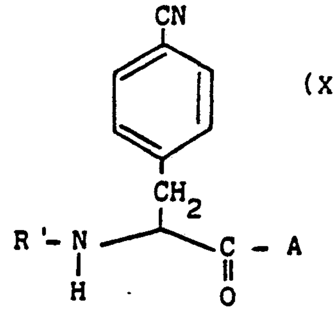

(X)

wherein R′ represents an N-protecting group and A represents the remainder of the coupling reagent, is reacted with the amine of formula

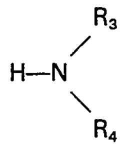

(XI)

wherein $R_3$ and $R_4$ have the same meanings as in formula II, to form the compound of formula:

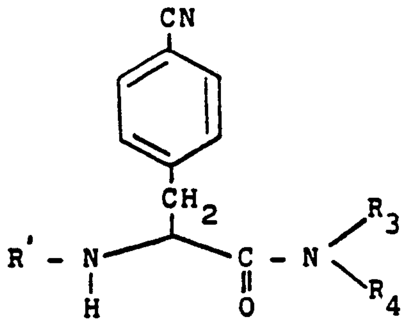

(XII)

wherein R′, $R_2$ and $R_4$ have the same meanings as in formulae II and IX, which is then reacted with a compound $R_2$—X, wherein X is a halogen and $R_2$ is as defined in formula II, to obtain the compound of formula

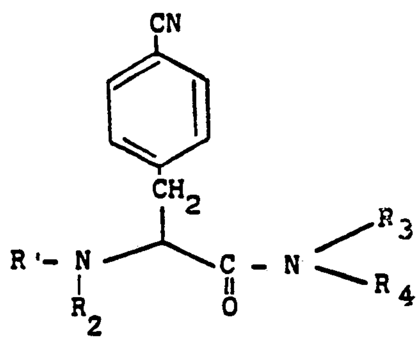

(XIII)

which, by cleaving of the protecting group R′, yields the compounds of formula (II).